# EUROPÄISCHE PATENTSCHRIFT

(11) **EP 2 678 322 B1**
(45) Veröffentlichungstag und Bekanntmachung des Hinweises auf die Patenterteilung: **19.07.2017**
(21) Anmeldenummer: 12701510.5
(22) Anmeldetag: 27.01.2012
(51) Int. Cl.: C07D 307/68, C08K 5/12

(54) **PENTYLESTER DER FURANDICARBONSÄURE ALS WEICHMACHER**
FURANDICARBONIC ACID PENTYL ESTER AS PLASTICIZERS
ESTERS D'ACIDE FURANDICARBOXYLIQUE COMME PLASTIFIANTS

(30) Priorität: 24.02.2011 DE 102011004676
(43) Veröffentlichungstag der Anmeldung: 01.01.2014
(73) Patentinhaber: Evonik Degussa GmbH, 45128 Essen (DE)
(72) Erfinder: BECKER, Hinnerk Gordon, 45257 Essen (DE); GRASS, Michael, 45721 Haltern am See (DE)
(86) Internationale Anmeldenummer: PCT/EP2012/051315
(87) Internationale Veröffentlichungsnummer: WO 2012/113608

(56) Entgegenhaltungen:
- WO-A1-2011/023590
- JP-A- 2008 127 282
- YODER P A ET AL: "Ueber Dehydroschleimsäure: eine neue Darstellungsmethode, sowie verschiedene Salze und Ester derselben", BERICHTE DER DEUTSCHEN CHEMISCHEN GESELLSCHAFT ABTEILUNG B:ABHANDLUNGEN, DE, Bd. 34, Nr. 3, 1. Oktober 1901 (1901-10-01), Seiten 3446-3462, XP008148998, ISSN: 0365-9488, DOI: 10.1002/CBER.19010340329 [gefunden am 2006-01-24]
- SANDERSON R D ET AL: "SYNTHESIS AND EVALUATION OF DIALKYL FURAN-2,5-DICARBOXYLATES AS PLASTICIZERS FOR PVC", JOURNAL OF APPLIED POLYMER SCIENCE, JOHN WILEY & SONS, INC, US, Bd. 53, Nr. 13, 26. September 1994 (1994-09-26), Seiten 1785-1793, XP000464476, ISSN: 0021-8995, DOI: 10.1002/APP.1994.070531308 in der Anmeldung erwähnt

## Beschreibung

Die vorliegende Erfindung betrifft die Verwendung von Pentylestern der Furandicarbonsäure als oder in Weichmachern sowie in Zusammensetzungen, insbesondere in solchen, die Polymere, insbesondere PVC enthalten. Weiterhin sind Gegenstand der Erfindung Kunststoffzusammensetzungen enthaltend diesen Pentylester sowie Formkörper oder Folien hergestellt aus diesen bzw. unter Verwendung dieser Zusammensetzungen.

Polyvinylchlorid (PVC) ist eines der wirtschaftlich bedeutendsten Polymere und wird sowohl als Hart-PVC wie auch als Weich-PVC in vielfältigen Anwendungen eingesetzt. Wichtige Anwendungsbereiche sind beispielsweise Kabelummantelungen, Fußbodenbeläge, Tapeten und Rahmen für Kunststofffenster. Zur Erhöhung der Elastizität und zur besseren Verarbeitbarkeit werden dem PVC Weichmacher zugesetzt. Zu diesen üblichen Weichmachern gehören beispielsweise Phthalsäureester wie Di-2-ethylhexylphthalat (DEHP), Disononylphthalat (DINP) und Diisodecylphthalat (DIDP). Wegen ihrer toxikologischen Eigenschaften ist man bemüht Phthalsäureester durch andere Weichmacher zu ersetzen. Als alternative Weichmacher sind daher in neuerer Zeit beispielsweise Cylclohexandicarbonsäureester beschrieben worden wie Di-isononylcyclohexancarbonsäureester (DINCH).

Weiterhin wurden im Stand der Technik auch Ester der Terephthalsäure als alternative Weichmacher beschrieben.

Bezüglich der Rohstoffbasis liegt die Besonderheit der vorliegenden Erfindung in der optionalen Nutzung nachwachsender Rohstoffe zur Herstellung der erfindungsgemäßen Furandicarbonsäureester. Dabei versteht man im Sinne der vorliegenden Erfindung unter nachwachsenden Rohstoffen im Unterschied zu petrochemischen Rohstoffen, welche auf fossilen Ressourcen, wie z.B. Erdöl oder Steinkohle basieren, solche Rohstoffe, die auf Basis von Biomasse entstehen bzw. hergestellt werden. Die Begriffe "Biomasse", "biobasiert" oder "basierend auf" bzw. "hergestellt aus nachwachsenden Rohstoffen" umfassen alle Materialien biologischen Ursprungs, die dem sogenannten "Kohlenstoff-Kurzzeitzyklus" entstammen, somit nicht Bestandteil geologischer Formationen oder Fossilschichten sind. Die Identifizierung und Quantifizierung nachwachsender Rohstoffe erfolgt gemäß ASTM-Methode D6866. Kennzeichnend ist u.a. für nachwachsende Rohstoffe ihr Anteil an dem Kohlenstoffisotop ¹⁴C im Gegensatz zu petrochemischen Rohstoffen.

Es ist bekannt, dass mit zunehmender Alkyl-Kettenlänge der Ester deren Unverträglichkeit mit Polymeren, insbesondere mit PVC ansteigt. Dies kann beispielsweise zur Konsequenz haben, dass PVC-Zusammensetzungen welche derartige Moleküle z.B. als Weichmacher enthalten, atypische und nicht vorhersehbare Viskositätsverläufe zeigen, die die Verarbeitung erschweren. Bei der Herstellung von Folien zeigt sich oftmals, dass diese zunehmend intransparent erscheinen, und eine Verfärbung der Folie eintritt, die sich beispielsweise in einem erhöhten Gelbwert widerspiegelt, der in den meisten Anwendungen unerwünscht ist. Durch eine geringere Verträglichkeit von Weichmachern und PVC wird auch die Permanenz des Weichmachers verringert, d. h., dass diese Weichmacher relativ schnell aus dem PVC-Halbzeug, Fertigzeug bzw. Produkt austreten, was zu einer Versprödung des Produktes führt und damit zu einer starken Funktions- und Wertminderung des entsprechenden Produktes. Man bezeichnet das Verhalten des Weichmachers auch als "Ausbluten" oder "Ausschwitzen".

Andererseits ist auch bekannt, dass Ester mit kurzen Alkylketten in der Regel einerseits eine höhere Flüchtigkeit aufweisen, andererseits aber auch, insbesondere wenn es sich um Ester mit hohem Geliervermögen handelt, bei der Verarbeitung in PVC-Pasten zu Pasten mit geringer Lagerstabilität führen deren Scherviskosität häufig sehr stark von der Schergeschwindigkeit abhängt, was wiederum zu einer eingeschränkten Verarbeitungsfähigkeit führt.

Bei der Herstellung von PVC-Plastisolen ist insbesondere darauf zu achten, dass bei der Verarbeitung eine möglichst niedrige Viskosität eingehalten wird, um eine gleichmäßige Verteilung des Weichmachers im PVC zu erreichen. Darüber hinaus sind auch eine hohe Lagerstabilität des PVC-Plastisols sowie eine geringe Abhängigkeit der Scherviskosität der Paste von der Schergeschwindigkeit erwünscht. Aus PVC-Plastisolen hergestellte (ungefüllte) Folien sollen transparent sein und einen möglichst niedrigen Gelbwert aufweisen. Der Weichmacher soll weiterhin eine hohe Permanenz aufweisen.

Im Stand der Technik sind verschiedene alternative Weichmacher bekannt geworden für den Einsatz in PVC. Die EP 1 808 457 A1 beschreibt den Einsatz von Terephthalsäuredialkylestern, die dadurch gekennzeichnet sind, dass die Alkylreste eine längste Kohlenstoffkette von mindestens 4 Kohlenstoffatomen aufweisen und eine Gesamtzahl an Kohlenstoffatomen pro Alkylrest von 5 aufweisen. Es wird weiterhin beschrieben, dass Terephthalsäureester mit 4 bis 5 Kohlenstoffatomen in der längsten Kohlenstoffkette des Alkohols als schnell gelierende Weichmacher für PVC gut geeignet sind.

Weiterhin ist auch die Verwendung von C5-Alkylestern der Citronensäure als Weichmacher mit guten Geliereigenschaften aus der EP 1864964B1 bekannt.

Viele der Ester der Furandicarbonsäure sind bei Raumtemperatur kristalline Feststoffe, die sich aufgrund ihrer Feststoffeigenschaft nur schwer für die Herstellung von flüssigen Zusammensetzungen, insbesondere von (Polymer-)Plastisolen einsetzen lassen. So ist die Herstellung von Polymerpasten bzw. Plastisolen im technischen Maßstab nur mit flüssigen Weichmachern zu realisieren. Feste Weichmacher müssen vorher in entsprechenden Lösungsmitteln gelöst werden, was das Verfahren aufwendig und teuer macht, und in vielen Anwendungen in Folge der Verdampfung der verwendeten Lösungsmittel während der Verarbeitung zur Problemen mit den Materialeigenschaften führt.

Die technische Aufgabe der Erfindung war es daher, Verbindungen zur Verfügung zu stellen, die als oder in Weichmachern eingesetzt werden können, welche sich auch in Plastisolen verarbeiten lassen, gute Geliereigenschaften besitzen, in Plastisolen eine geringe Abhängigkeit der Plastisolviskosität von der Schergeschwindigkeit zeigen und einen niedrigen Gelbwert und hohe Transparenz bei der Verarbeitung zu Folien aufweisen. Eine zusätzliche Aufgabe war es die Lösung dieser technischen Problemstellung in Zusammenhang mit einer Substanz bzw. Verbindung zu lösen, welche zumindest teilweise aus nachwachsenden Rohstoffen hergestellt werden kann.

Diese technische Aufgabe wird gelöst durch die Verwendung von Dipentylestern der Furandicarbonsäure, deren Eigenviskosität bei 25 °C maximal 60 mPa*s beträgt, als Weichmacher.

Insbesondere wird die technische Aufgabe gelöst durch Pentylester der Furandicarbonsäure welche mindestens eine der folgenden Eigenschaften aufweisen:
- die Dichte bei 20 °C beträgt maximal 1,06 g/cm³;
- beim Vermessen mit einem Differentialkalorimeter tritt kein Schmelzsignal bei Temperaturen > 25 °C auf.

In einer weiteren Ausführungsform weist der Pentylester mindestens zwei der zuvor genannten Eigenschaften auf.

Ein besonderer ökonomischer und gleichzeitig ökologischer Vorteil der vorliegenden Erfindung liegt in der gleichzeitigen Nutzung von nachwachsenden und petrochemischen Rohstoffen für die Herstellung der erfindungsgemäßen Furandicarbonsäureester, was einerseits eine besonders preiswerte Herstellung und eine breite Anwendbarkeit ermöglicht, andererseits aber auch zu besonders "nachhaltigen" Produkten führt.

Überraschenderweise wurde festgestellt, dass solche Pentylester im Gegensatz zu den entsprechenden homologen Butyl- und Hexylestern keine Feststoffe sind, und als Flüssigkeiten bei Raumtemperatur gut zu verarbeiten sind. Die entsprechenden homologen Di-n-butylfurandicarboxylate und Di-n-hexylfurandicarboxylate sind bei Raumtemperatur fest und besitzen Schmelzpunkte im Bereich von 30-40 °C. Diese können daher nicht im technischen Maßstab zur Herstellung von Polymerpasten bzw. Plastisolen eingesetzt werden.

Di-n-butyl-furandicarboxylat und Di-n-hexyl-furandicarboxylat sind aus den Arbeiten von Sanderson et al (R. D. Sanderson, D.F.Schneider, I. Schreuder; J.Appl.Polym.Sci.; 53 (1991); 1785-1793) bekannt. Es handelt sich dabei um kristalline Feststoffe mit Schmelzpunkten von ca. 42 °C (Di-n-butyl-furandicarboxylat) und ca. 32 °C (Di-n-hexyl-furandicarboxylat), die für zahlreiche Anwendungen wie z.B. die Herstellung von Polymerpasten nicht sinnvoll einsetzbar sind.

Dipentylester der Furandicarbonsäure sind bislang noch nicht als beschrieben worden, insbesondere gibt es keine Hinweise zur Verwendung von Dipentylestern der Furandicarbonsäure in Polymerzusammensetzungen und/oder als Weichmacher.

Überraschenderweise wurde nun gefunden, dass die erfindungsgemäßen Pentylester in flüssiger Form ohne Verfestigung hergestellt werden können, und sich vorteilhaft als Komponente in Polymerformulierungen, z.B. als Weichmacher in PVC-Formulierungen einsetzen lassen.

Es wurde weiterhin festgestellt, dass die erfindungsgemäßen Pentylester exzellente Geliereigenschaften aufweisen, wenn sie mit PVC verarbeitet werden. Diese Geliereigenschaften sind sogar besser, als die Geliereigenschaften der entsprechenden Terephthalsäuredialkylester.

Die PVC-Pasten auf Basis der erfindungsgemäßen Pentylester weisen nur eine geringe Abhängigkeit der Pastenviskosität von der Schergeschwindigkeit auf. Sie sind damit in einem weiten Schergeschwindigkeitsbereich und mit unterschiedlichsten Verarbeitungsmethoden verarbeitbar.

Aufgrund des günstigen Gelierverhaltens können die entsprechenden PVC-Pasten schneller bzw. bei niedrigeren Temperaturen verarbeitet werden.

Es wurde weiterhin festgestellt, dass transparente Folien aus PVC, die den erfindungsgemäßen Weichmacher enthalten, eine sehr hohe Transparenz aufweisen, die zum Teil höher ist als von Folien, die mit Dibutylterephthalat als Weichmacher hergestellt wurden.

In einer bevorzugten Ausführungsform ist der Pentylester ein Dipentylfuran-2,5-dicarboxylat. Dieses kann auch in Form von mindestens zwei isomeren Dipentylfuran-2,5-dicarboxylaten vorhanden sein.

In einer bevorzugten Ausführungsform werden auch Ester eingesetzt aus isomeren Dipentylfuran-2,5-dicarboxylaten, wobei diese Pentylgruppen enthalten, die ausgewählt sind aus: n-Pentyl-, 2-Pentyl-, 3-Pentyl-, 2-Methylbutyl-, 3 Methylbutyl-, 3-Methylbut-2-yl-, 2-Methylbut-2-yl-Gruppen.

Besonders bevorzugt werden Gemische aus n-Pentanol und 2-Methylbutanol im MassenVerhältnis von 99,9 bis 70 % Pentanol und 0,1 bis 30 % 2-Methylbutanol eingesetzt.

Die Alkylreste der Furandicarbonsäureester sind vorzugsweise zu mehr als 60 % n-Pentyl-Reste. Bevorzugt sind die Alkylreste der Terephthalsäuredialkylester von 70 bis 99,9 % n-Pentyl-Reste und von 30 bis 0,1 % Methylbutyl-Reste, insbesondere 2-Methylbutylreste, besonders bevorzugt von 85 bis 98 % n-Pentyl-Reste und von 15 bis 2 % Methylbutyl-Reste, insbesondere 2-Methylbutylreste, und ganz besonders bevorzugt von 90 bis 96 % n-Pentyl-Reste und von 10 bis 4 % Methylbutyl-Reste, insbesondere 2-Methylbutylreste. Vorzugsweise sind die Methylbutyl-Reste zu mehr als 50 %, bevorzugt zu mehr als 75 % und besonders bevorzugt zu mehr als 95 % 2-Methylbutyl-Reste. Die Bestimmung der prozentualen Verteilung der C₅-Alkyl-Reste kann auf einfache Weise durch Verseifen der Ester, Abtrennen des erhaltenen Alkohols und gaschromatographische (GC) Analyse des Alkohols erfolgen. Beispielsweise kann die gaschromatographische Trennung auf einer Polydimethylsiloxan-Säule (z. B. DB 5) als stationärer Phase mit einer Länge von 60 m, einem inneren Durchmesser von 0,25 mm und einer Filmdicke von 0,25 µm durchgeführt werden.

In einer weiteren besonderen, bevorzugten Ausführungsform handelt es sich bei dem erfindungsgemäßen Pentylester um einen Di-n-pentylester. Dies hat den Vorteil, dass es sich um eine Verbindung mit eindeutiger Molekülstruktur handelt, die unproblematisch aus dem industriell verfügbaren Rohstoff n-Pentanol hergestellt werden kann.

In einer weiteren besonderen, bevorzugten Ausführungsform handelt es sich bei mindestens einer der beiden Estergruppen des erfindungsgemäßen Pentylesters um eine 2- Methylbutyl- oder 3-Methylbutylgruppe. Dies hat den Vorteil, dass die Pentylester eine besonders hohe Lagerstabilität sowohl an sich als auch in Polymerzusammensetzungen aufweisen.

In einer weiteren bevorzugten Ausführungsform handelt es sich bei den erfindungsgemäßen Pentylestern um Estergemische isomerer Pentanole, Dies hat den Vorteil, dass hierbei Gemische mit sehr geringer Kristallisationsneigung hergestellt werden können, die in Polymerzusammensetzungen zu guten Tieftemperatureigenschaften führen.

Bevorzugt können zur Herstellung der erfindungsgemäßen Pentylester primäre Alkohole oder Alkoholgemische wie sie beispielsweise durch Hydroformylierung eines Alkens mit nachfolgender Hydrierung erhalten werden können, eingesetzt werden. Vorstufen für Pentanole sind vorzugsweise technische Kohlenwasserstoffgemische, die ein oder mehrere Olefin(e) mit 4 Kohlenstoffatomen enthalten. Die bedeutendste Quelle für C₄-Olefine ist der C₄-Schnitt des Crackbenzins aus Steamcrackern. Daraus wird nach Extraktion(sdestillation) des Butadiens oder dessen Selektivhydrierung zu einem n-Butengemisch ein Kohlenwasserstoffgemisch (Raffinat I oder hydriertes Crack-C₄) hergestellt, das Isobuten, 1-Buten und die beiden 2-Butene enthält. Ein anderer Rohstoff für C₄-Olefine ist der C₄-Schnitt aus FCC-Anlagen, der wie oben beschrieben aufgearbeitet werden kann. C₄-Olefine, hergestellt durch Fischer-Tropsch-Synthese, sind nach Selektivhydrierung des darin enthaltenen Butadiens zu n-Butenen ebenfalls ein geeigneter Einsatzstoff. Darüber hinaus können Olefingemische, die durch Dehydrierung von C₄-Kohlenwasserstoffen oder durch Metathesereaktionen erhalten werden, oder andere technische Olefinströme geeignete Einsatzstoffe sein. Neben dem Raffinat I sind als Vorstufen für die Pentanole auch Raffinat II und/oder Raffinat III, ein Strom welcher durch Abtrennung des größten Teils an 1-Buten aus dem Raffinat II erhalten wird, und sogenanntes Rohbutan, welches nach einer Oligomerisierung von Raffinat II anfällt und welches neben Alkanen als Olefin praktisch ausschließlich 2-Buten aufweist, geeignet. Der Vorteil der Verwendung von Raffinat II, Raffinat III oder Rohbutan als Vorstufe für Pentanole liegt darin, dass diese Vorstufen kein bzw. nahezu kein Isobuten aufweisen, weshalb die erhaltenen Pentanole kein bzw. nur geringe Mengen (kleiner 0,5 Massen-% in Bezug auf die Pentanole) an 3-Methylbutanol aufweisen.

Wegen des häufig sehr hohen Trennaufwands zur Trennung der Einsatzgemische kann es vorteilhaft sein, die im als Einsatzgemisch einzusetzenden technischen Gemisch vorliegenden Olefine nicht zu trennen, sondern die Gemische direkt einzusetzen.

In den erfindungsgemäßen Zusammensetzungen können vorzugsweise weitere zusätzliche Weichmacher, ausgenommen Pentylester der Furandicarbonsäure enthalten sein.

Solche zusätzlichen Weichmacher sind beispielsweise ausgewählt aus folgender Liste: Phthalsäuredialkylester, bevorzugt mit 4 bis 13 C-Atomen in der Alkylkette; Trimellitsäuretrialkylester, bevorzugt mit 4 bis 9 C-Atomen in der Seitenkette; Adipinsäuredialkylester, bevorzugt mit 4 bis 9 C-Atomen in der Seitenkette; Terephthalsäuredialkylester, jeweils bevorzugt mit 4 bis 13 C-Atomen, insbesondere 4 bis 9 C-Atomen in der Seitenkette; 1,2-Cyclohexandisäurealkylester, 1,3-Cyclohexandisäurealkylester und 1,4-Cyclohexandisäurealkylester, hierbei bevorzugt 1,2-Cyclohexandisäurealkyl-ester, jeweils bevorzugt mit 4 bis 10 Kohlenstoffatomen in der Seitenkette; Dibenzoesäurester von Glykolen; Alkylsulfonsäureester von Phenol mit vorzugsweise einem Alkylrest, der 8 bis 22 C-Atome enthält; Glycerinester; Isosorbidester, insbesondere Isosorbiddiester; epoxidierte Pflanzenöle; gesättigte oder ungesättigte Fettsäureester, welche auch partiell oder vollständig epoxidiert sein können; Citronensäuretriester mit freier oder carboxylierter OH-Gruppe und beispielsweise Alkylresten von 4 bis 8 C-Atomen, Alkylpyrrolidonderivate mit Alkylresten von 4 bis 18 C-Atomen sowie Benzoesäurealkylester, vorzugsweise mit 7 bis 13 C-Atomen in der Alkylkette. In allen Fällen können die Alkylreste linear oder verzweigt und gleich oder verschieden sein.

Besonders bevorzugt wird in den erfindungsgemäßen Mischungen kein *ortho*-Phthalat als zusätzlicher Weichmacher eingesetzt.
In einer besonderen Ausführungsform handelt es sich bei mindestens einem der in der erfindungsgemäßen Zusammensetzung verwendeten zusätzlichen Weichmacher, um ein Trimellitsäuretrialkylester. Bevorzugt weist dieser Trimellitsäuretrialkylester Esterseitenketten mit 4 bis 9 Kohlenstoffatomen auf, wobei die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 8 Kohlenstoffatomen pro Estergruppe, insbesondere bevorzugt um eine Gruppe mit maximal 7 Kohlenstoffatomen und ganz besonders bevorzugt um eine Gruppe mit maximal 6 Kohlenstoffatomen. Die Kombination der erfindungsgemäßen Pentylester mit Trimellitsäuretrialkylestern führt bei Verwendung in PVC-Plastisolen insbesondere zu Produkten, die einen niedrigen Anteil an flüchtigen Bestandteilen und gute Temperaturbeständigkeit aufweisen.

In einer weiteren besonderen Ausführungsform handelt es sich bei mindestens einem der in der erfindungsgemäßen Zusammensetzung verwendeten zusätzlichen Weichmacher, um ein Adipinsäuredialkylester. Bevorzugt weist dieser Adipinsäuredialkylester Esterseitenketten mit 4 bis 9 Kohlenstoffatomen auf, wobei auch hier die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 8 Kohlenstoffatomen pro Estergruppe, insbesondere bevorzugt um eine Gruppe mit maximal 7 Kohlenstoffatomen. Insbesondere handelt es sich bei mindestens einem der verwendeten Adipinsäuredialkylester um Dioctyladipat. Die Kombination der erfindungsgemäßen Furandicarbonsäuredipentylester mit Adipinsäuredialkylestern führt bei Verwendung in PVC-Plastisolen insbesondere zu Produkten, die eine niedrige Plastisolviskosität sowie im verarbeiteten Zustand gute Tieftemperatureigenschaften (z.B. eine sehr niedrige Glasübergangstemperatur) aufweisen.

In einer weiteren besonderen Ausführungsform handelt es sich bei mindestens einem der in der erfindungsgemäßen Zusammensetzung verwendeten zusätzlichen Weichmacher, um ein Terephthalsäuredialkylester. Bevorzugt weist dieser Terephthalsäuredialkylester Esterseitenketten mit 4 bis 13 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 10 Kohlenstoffatomen pro Estergruppe, insbesondere bevorzugt um eine Gruppe mit maximal 9 Kohlenstoffatomen und ganz besonders bevorzugt um eine Gruppe mit maximal 8 Kohlenstoffatomen. Insbesondere handelt es sich bei mindestens einem der verwendeten Terephthalsäuredialkylester um Di-(isononyl)terephthalat, Di-(2-ethylhexyl)terephthalat, Di-*n*-heptylterephthalat, Di-*iso*-heptylterephthalat, Di-*n-*butylterephthalat, Di-(3-methylbutyl)terephthalat oder Di-*n*-pentylterephthalat. Die Kombination der erfindungsgemäßen Furandicarbonsäuredipentylester mit Terephthalsäuredialkylestern führt bei Verwendung in PVC-Plastisolen insbesondere zu Produkten, die (je nach Esterkettenlänge der verwendeten Terephthalsäuredialkylester) eine sehr gute Thermostabilität und gute Tieftemperatureigenschaften bei gleichzeitig geringen flüchtigen Bestandteilen aufweisen.

In einer weiteren besonderen Ausführungsform handelt es sich bei mindestens einem der in der erfindungsgemäßen Zusammensetzung verwendeten zusätzlichen Weichmacher, um einen Dialkylester der Cyclohexandicarbonsäure, besonders bevorzugt um einen Dialkylester der 1,2-Cyclohexandicarbonsäure. Bevorzugt weist dieser Cyclohexandicarbonsäuredialkylester Esterseitenketten mit 4 bis 10 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 9 Kohlenstoffatomen pro Estergruppe, insbesondere bevorzugt um eine Gruppe mit maximal 8 Kohlenstoffatomen und ganz besonders bevorzugt um eine Gruppe mit maximal 7 Kohlenstoffatomen. Insbesondere handelt es sich bei mindestens einem der verwendeten Cyclohexandicarbonsäuredialkylester um 1,2-Cyclohexansäuredi-*iso-*nonylester, 1,2-Cyclohexansäuredi-2-ethylhexylester, 1,2-Cyclohexansäuredi-*n*-pentylester, 1,2-Cyclohexan-säuredi-*n*-heptylester, 1,2-Cyclohexansäuredi-*iso*-heptylester, 1,2-Cyclohexansäuredi-*n*-butylester, 1,4-Cyclohexansäuredi-*n*-butylester, 1,3-Cyclohexansäuredi-*n-*butylester oder 1,2-Cyclohexansäuredi(-3-methylbutyl)ester. Die Kombination der erfindungsgemäßen Furandicarbonsäuredipentylester mit Dialkylestern der Cyclohexandicarbonsäure führt bei Verwendung in PVC-Plastisolen insbesondere zu Produkten, die sich insbesondere durch eine sehr hohe Hydrolysebeständigkeit und eine sehr niedrige Plastisolviskosität bei gleichzeitig guten Geliereigenschaften auszeichnen.

In einer weiteren besonderen Ausführungsform handelt es sich bei mindestens einem der in der erfindungsgemäßen Zusammensetzung verwendeten zusätzlichen Weichmacher, um einen Glycerinester, besonders bevorzugt um einen Glycerintriester. Die Estergruppen können dabei sowohl von aliphatischer als auch aromatischer Struktur sein. Bevorzugt weist dieser Glycerinester Esterseitenketten mit 1 bis 24 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei einer der Estergruppen um Hydroxystearinsäure, wobei die Hydroxyfunktion bevorzugt ebenfalls verestert ist, besonders bevorzugt durch eine Acetylgruppe Weiterhin besonders bevorzugt handelt es sich bei mindestens einer der vorhandenen Estergruppen um eine Gruppe mit maximal 9 Kohlenstoffatomen pro Estergruppe, insbesondere bevorzugt um eine Gruppe mit maximal 8 Kohlenstoffatomen und ganz besonders bevorzugt um eine Gruppe mit maximal 7 Kohlenstoffatomen. Insbesondere handelt es sich bei mindestens einem der verwendeten Glycerinester um ein Glycerintriacetat. Die Kombination der erfindungsgemäßen Furandicarbonsäuredipentylester mit Glycerinestern führt insbesondere zu besonders nachhaltigen Produkten welche zu einem großen Anteil auf Basis nachwachsender Rohstoffe hergestellt werden können.

In einer weiteren besonderen Ausführungsform handelt es sich bei mindestens einem der in der erfindungsgemäßen Zusammensetzung verwendeten zusätzlichen Weichmacher, um einen Citronensäuretriester mit freier oder carboxylierter OH-Gruppe. Die Estergruppen können dabei auch hier sowohl von aliphatischer als auch aromatischer Struktur sein. Insbesondere bevorzugt handelt es sich um einen Citronensäuretrialkylester mit carboxylierter OH-Gruppe. Bevorzugt weist dieser Citronensäuretrialkylester Esterseitenketten mit 1 bis 9 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit maximal 9 Kohlenstoffatomen pro Estergruppe, insbesondere bevorzugt um eine Gruppe mit maximal 8 Kohlenstoffatomen und ganz besonders bevorzugt um eine Gruppe mit maximal 7 Kohlenstoffatomen. Insbesondere handelt es sich bei mindestens einem der verwendeten Citronensäureestern um Acetyl-tributylcitrat, Acetyl-tri-*n*-butylcitrat, Acetyl-tri-*n*-pentylcitrat oder Acetyl-tri-*iso*-heptylcitrat. Die Kombination der erfindungsgemäßen Furandicarbonsäuredipentylester mit Citronensäuretriestern mit freier oder carboxylierter OH-Gruppe führt insbesondere zu Plastisolen, die eine besonders gute Gelierfähigkeit insbesondere bei niedrigen Temperaturen besitzen.

In einer bevorzugten Ausführungsform liegt das Massenverhältnis aus eingesetzten zusätzlichen Weichmachern und Pentylestern der Furandicarbonsäure zwischen 1:20 und 20:1, bevorzugt zwischen 1:10 und 20:1, besonders bevorzugt zwischen 1:5 und 20:1 und insbesondere besonders bevorzugt zwischen 1:1 und 15:1.

Die erfindungsgemäßen Pentylester bzw. die aus ihnen hergestellten Weichmacher können in allen möglichen Darreichungsformen vorliegen, z.B. als Flüssigkeit, insbesondere als (bei

Raumtemperatur) pumpbare Flüssigkeit, als Paste, Schutzmasse, Plastisol, Pulver, Feststoff oder Festkörper. Insbesondere bevorzugt liegen sie als Flüssigkeit und insbesondere bevorzugt als (bei Raumtemperatur) pumpbare Flüssigkeit vor.

Beansprucht wird die Verwendung der Dipentylester als Weichmacher für Polymere. Bei dem Polymer handelt es sich vorzugsweise um PVC.

Der erfindungsgemäße Pentylester kann besonders vorteilhaft in Klebstoffen, Dichtungsmassen, Lacken, Farben, Plastisolen, Kunstleder, Fußbodenbeläge, Unterbodenschutz, Gewebebeschichtungen, Tapeten oder Tinten verwendet werden.

Des Weiteren werden Kunststoffzusammensetzungen, welche einen zuvor beschriebenen Weichmacher enthalten, beansprucht.

Die erfindungsgemäßen Weichmacher können in Zusammensetzungen eingesetzt werden, die Polymere enthalten. Diese Polymere sind insbesondere ausgewählt aus der Gruppe bestehend aus: Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalkohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat-Copolymere (A-SA), Styrolacrylonitril-Copolymere (SAN), Acrylonitril-Butadien-Styrol-Copolymere (ABS), Styrol-Maleinsäureanhydrid-Copolymere (SMA), Styrol-Methacrylsäure-Copolymere, Polyolefine und/oder Polyolefincopolymere, insbesondere Polyethylen (PE) oder Polypropylen (PP), thermoplastische Polyolefine (TPO), Polyethylen-Vinylacetat-Copolymere (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten aufweisen. Vorzugsweise enthalten die erfindungsgemäßen Polymerzusammensetzungen PVC oder Homo- oder Copolymere auf Basis von Ethylen, Propylen, Butadien, Vinylacetat, Glycidylacrylat, Glycidylmethacrylat, Methacrylaten, Ethylacrylaten, Butylacrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf.

Besonders bevorzugt enthalten die erfindungsgemäßen Kunststoffzusammensetzungen als PVC-Typ Suspensions-, Masse-, Mikrosuspensions- oder Emulsions-PVC.

Bezogen auf 100 Massenteile Polymer enthalten die erfindungsgemäßen Kunststoffzusammensetzungenvon 5 bis 200, bevorzugt von 10 bis 150 Massenteilen an Weichmacher.

Die erfindungsgemäßen Kunststoffzusammensetzungen können neben den genannten Bestandteilen weitere Bestandteile enthalten, die insbesondere ausgewählt sind aus der Gruppe bestehend aus Füllstoffen, Pigmenten, Thermostabilisatoren, Costabilisatoren, Antioxidantien, Viskositätsregulierer und Gleitmittel.

Thermostabilisatoren neutralisieren beispielsweise während und/oder nach der Verarbeitung von PVC abgespaltene Salzsäure und verhindern einen thermischen Abbau des Polymeren. Als Thermostabilisatoren kommen alle üblichen Stabilisatoren in fester und flüssiger Form in Betracht, beispielsweise auf Basis von Ca/Zn, Ba/Zn, Pb, Sn oder organischer Verbindungen (OBS), sowie auch säurebindende Schichtsilikate wie Hydrotalcit. Die erfindungsgemäßen Gemische können einen Gehalt von 0,5 bis 10, bevorzugt 1 bis 5, besonders bevorzugt von 1,5 bis 4 Massenteilen pro 100 Massenteilen Polymer an Thermostabilisator aufweisen.
Als sogenannte Costabilisatoren (d.h. Substanzen, die die Wirkung der Thermostabilisatoren verlängern, verbessern und/oder ergänzen), können z.B. Pflanzenölderivate wie z.B. epoxidiertes Sojabohnenöl oder epoxidiertes Leinöl eingesetzt werden.

Als Pigmente können im Rahmen der vorliegenden Erfindung sowohl anorganische als auch organische Pigmente eingesetzt werden. Der Gehalt an Pigmenten liegt zwischen 0,01 bis 10 Massen-%, bevorzugt 0,05 bis 5 Massen-%, besonders bevorzugt 0,1 bis 3 Massen-% pro 100 Massenteilen Polymer. Beispiele für anorganische Pigmente sind TiO₂, CdS, CoO/Al₂O₃, Cr₂O₃. Bekannte organische Pigmente sind beispielsweise Azofarbstoffe, Phthalocyaninpigmente, Dioxazinpigmente sowie Anilinpigmente.

Die erfindungsgemäßen Kunststoffzusammensetzungen können alle dem Stand der Technik entsprechenden Füllstoffe enthalten. Beispiele solcher Füllstoffe sind mineralische und/oder synthetische und/oder natürliche, organische und/oder anorganische Materialien, wie z. B. Calciumoxid, Magnesiumoxid, Calciumcarbonat, Bariumsulfat, Siliziumdioxid, Schichtsilikat, Industrieruß, Bitumen, Holz (z. B. pulverisiert, als Granulat, Mikrogranulat, Fasern usw.), Papier, Natur- und/oder Synthetikfasern usw.. Besonders bevorzugt handelt es sich bei mindestens einem der eingesetzten Füllstoffe um ein Calciumcarbonat oder ein Calcium-Magnesium-Carbonat.

Als viskositätssenkende Reagenzien können aliphatische oder aromatische Kohlenwasserstoffe aber auch Alkohole und/oder Carbonsäurederivate wie beispielsweise 2,2,4-Trimethyl-1,3-pentandiol-diisobutyrat, eingesetzt werden. Viskositätssenkende Reagenzien werden den erfindungsgemäßen Zusammensetzungen insbesondere in Anteilen von 0,5 bis 50, bevorzugt 1 bis 30, besonders bevorzugt 2 bis 10 Massenteilen pro 100 Massenteilen Polymer zugegeben.

Ein weiterer Gegenstand der Erfindung sind Formkörper oder Folien hergestellt aus bzw. enthaltend eine erfindungsgemäßen Kunststoffzusammensetzung.

Diese Formkörper oder Folien sind bevorzugt ein Fußbodenbelag, ein Wandbelag, ein Film / eine Folie, ein Schlauch, ein Profil, eine Dachbahn, eine Dichtungsbahn, eine Kabel- und Drahtummantelung, eine Plane, ein Werbebanner, Kunstleder, Verpackungsfolie, ein medizinischer Artikel, Spielzeug, eine Dichtung oder ein Einrichtungsgegenstand

Die erfindungsgemäßen Pentylester besitzen in ihrer Verwendung als Weichmacher zahlreiche Vorteile gegenüber bekannten Weichmachern des Standes der Technik. So sind diese Verbindungen überraschenderweise im Gegensatz zu den homologen Di-n-butylfurandicarboxylaten und Di-n-hexylfurandicarboxylaten, welche beide als kristalliner Feststoff mit Schmelzpunkten weit oberhalb der Raumtemperatur vorliegen, gut zu verarbeitende (u.a. gut zu dosierende) Flüssigkeiten.

Die Herstellung von flüssigen Zusammensetzungen, insbesondere Polymerzusammensetzungen wie Polymerpasten und Plastisolen im technischen Maßstab ist am besten mit flüssigen Weichmachern zu realisieren, da hier der Einsatz von Lösungsmitteln nicht bzw. nur in stark reduzierter Form notwendig ist. Lösemittel führen in der Regel auch zu einer "Verdünnung" des weichmachenden Effektes und müssen daher während der Herstellung wieder entfernt werden.

Da dies in den seltensten Fällen quantitativ geschehen kann, sind hohe flüchtige (organische) Anteile sog. "VOC's" in den Halb- bzw. Fertigprodukten enthalten, die wiederum prohibitiv für den Einsatz solcher Produkte insbesondere im Innerraumbereich sowie in Fahrzeugen sind. Hinzu kommt die Gefahr des Auskristallisierens von festen Weichmachern im Halb- bzw. Fertigprodukt was zu einer starken Verschlechterung der Materialeigenschaften, bis hin zum Materialversagen führt. Die alternativ zur Verwendung von Lösungsmitteln existierende Problemlösung, feste Weichmacher in geschmolzenem Zustand (d.h. bei erhöhter Temperatur) einzusetzen, ist in vielen Fällen technisch nicht umsetzbar. Auch besteht die Gefahr einer thermischen Schädigung von anderen Formulierungsbestandteilen.

Die Kristallinität der erfindungsgemäßen Ester sowie die Lage und Art der Schmelzpunktsignale (Peak) und Glasübergangssignale (Stufe) im DSC-Thermogramm, hängt vom Verzweigungsgrad der Esterketten und der Zusammensetzung der Ester ab, und ist über die zur Esterherstellung verwendeten Alkohole einstellbar. Beim der Vermessung der erfindungsgemäßen Pentylester (Reinheit It. GC-Analyse mindestens 98 Flächen%) in einem Differentialkalorimeter (DSC) tritt nach Abkühlung auf -150 °C in der ersten Aufheizung kein Schmelzpunkt bei Temperaturen oberhalb von 25 °C auf, bevorzugt kein Schmelzpunkt bei Temperaturen oberhalb von 22 °C, besonders bevorzugt kein Schmelzpunkt bei Temperaturen oberhalb von 18 °C und insbesondere bevorzugt kein Schmelzpunkt bei Temperaturen oberhalb von 16 °C.

In einer besonderen Ausführungsform wird bei der beschriebenen Vermessung mittels DSC eine Glasübergangstemperatur bei < 0 °C detektiert, bevorzugt bei < -10 °C, besonders bevorzugt bei < -20 °C und insbesondere bevorzugt bei < -30 °C.

Die Scherviskosität (Eigenviskosität) der erfindungsgemäßen Ester hängt ebenfalls vom Verzweigungsgrad der Esterketten und der Zusammensetzung der Ester ab, und ist über die zur Esterherstellung verwendeten Alkohole einstellbar. Die bei 20 °C bestimmte Scherviskosität der flüssigen erfindungsgemäßen Pentylester (Reinheit It. GC-Analyse mindestens 98 Flächen%) hängt vom Verzweigungsgrad der Esterketten und der Zusammensetzung der Ester ab. Sie liegt insbesondere bei maximal 60 mPa*s, bevorzugt bei maximal 55 mPa*s, besonders bevorzugt bei maximal 50 mPa*s und insbesondere bevorzugt bei unter 46 mPa*s.

Die Dichte der erfindungsgemäßen Ester hängt ebenfalls vom Verzweigungsgrad der Esterketten und der Zusammensetzung der Ester ab, und ist über die zur Esterherstellung verwendeten Alkohole einstellbar. Die bei 20 °C bestimmte Dichte der erfindungsgemäßen Ester (Reinheit It. GC-Analyse min. 99 FI.%) beträgt insbesondere maximal 1,06 g/cm³, bevorzugt maximal 1,05 g/cm³, besonders bevorzugt maximal 1,03 g/cm³ und insbesondere besonders bevorzugt maximal 1,01 g/cm³.

Ein weiterer Vorteil ist es, dass die erfindungsgemäßen Pentylester ein hervorragendes Geliervermögen für Polymere, insbesondere für PVC aufweisen und überraschender Weise auch eine deutlich geringere Lösetemperatur für PVC besitzen als beispielsweise Di-n-butylfurandicarboxylat. PVC-Pasten auf Basis der erfindungsgemäßen Furandicarbonsäureester weisen weiterhin auch eine deutlich geringere Geliertemperatur auf, als beispielsweise die aus dem industriellen Gebrauch bekannten Dibutylterephthalatpasten, obwohl die erfindungsgemäßen Ester längere Esterseitenketten als Dibutylterephthalat aufweisen. Sie können somit schneller und bei niedrigeren Temperaturen verarbeitet werden.

PVC-Plastisole / PVC-Pasten welche die erfindungsgemäßen Pentylester in einem Anteil von mindestens 10 ma% bezogen auf die insgesamt verwendeten Weichmacher enthalten, erreichen (Abhängig von Art und Menge der zusätzlich eingesetzten Weichmacher und Lösungsmittel) im mittels oszillierender Rheometrie durchgeführten Gelierversuch mit dynamischer Temperaturführung (konstante Heizrate) insbesondere eine Pastenviskosität von > 1.000 Pa*s bei Temperaturen von maximal 100 °C, bevorzugt von maximal 95 °C, besonders bevorzugt von maximal 90 °C, insbesondere bevorzugt von maximal 85 °C und insbesondere besonders bevorzugt von maximal 82 °C.

Die Temperatur bei der im oben beschriebenen Gelierversuch die maximale Viskosität erreicht wird, liegt insbesondere bei maximal 130 °C, bevorzugt bei maximal 120 °C, besonders bevorzugt bei maximal 118 °C, insbesondere bevorzugt bei maximal 115 °C und insbesondere besonders bevorzugt bei maximal 112 °C.

Es ist weiterhin festzustellen, dass PVC-Pasten auf Basis eines erfindundungsgemäßen Pentylesters eine geringe Abhängigkeit der Pastenviskosität von der Schergeschwindigkeit aufweisen als vergleichbare Pasten. Damit sind diese Polymerzusammensetzungen in einem weiten Schergeschwindigkeitsbereich und mit unterschiedlichsten Verarbeitungsmethoden verwendbar.

Es wurde weiterhin auch überraschenderweise festgestellt, dass ungefüllte, transparente PVC-Folien enthaltend einen erfindungsgemäßen Pentylester als Weichmacher eine sehr niedrige Opazität und damit eine hohe Transparenz aufweisen. Diese liegt zum Teil deutlich niedriger als bei Folien die auf Basis von Standardweichmachern hergestellt werden oder bei denen Dibutylterephthalat als Weichmacher eingesetzt wird.

Insbesondere werden bei der Herstellung transparenter Folien (Foliendicke 0,9 - 1,1 mm) aus PVC-Plastisolen / PVC-Pasten welche einen erfindungsgemäßen Pentylester in einem Anteil von mindestens 10 ma% bezogen auf die insgesamt verwendeten Weichmacher enthalten, Opazitätswerte von maximal 15 erzielt, bevorzugt maximal 14, besonders bevorzugt maximal 13, insbesondere bevorzugt maximal 12 und insbesondere besonders bevorzugt maximal 11. Der Gelbwert (Index YD 1925) der oben beschriebenen transparenten Folien liegt insbesondere bei maximal 18, bevorzugt bei maximal 16, besonders bevorzugt bei maximal 15 und insbesondere besonders bevorzugt bei maximal 14.

Die folgenden Beispiele sollen die Erfindung erläutern, ohne ihre Anwendungsbreite einzuschränken, die sich aus der Beschreibung und den Ansprüchen ergibt.

### BEISPIELE

### Beispiel 1

### Herstellung von Furan-2,5-dicarbonsäuredichlorid (II)

Die erfindungsgemäßen Ester wurden in einer zweistufigen Synthese ausgehend von Furan-2,5-dicarbonsäure über das Dichlorid hergestellt. In einem 250 mL-Dreihalskolben, mit Rückflusskühler und Tropftrichter wurden unter Argon 72,1 g (462 mmol) Furan-2,5-dicarbonsäure vorgelegt. In einem Zeitraum von 10 min wurden 165 g (1.39 mol) Thionylchlorid, versetzt mit einigen Tropfen N,N-Dimethylformamid, zugegeben. Die Suspension wurde auf Rückfluss-Temperatur erhitzt und das entstehende Gas durch Waschflaschen mit wässriger KOH-Lösung abgeleitet. Es wurde dann 4 h bis zur Beendigung der Gasentwicklung und vollständigen Auflösung des Feststoffes unter Rückfluss erhitzt. Die Isolierung des Produktes erfolgte, nach Abziehen von überschüssigem Thionylchlorid, durch destillative Aufreinigung (T = 110 °C, p = 0,0012 MPa). Hierbei resultierten 79,4 g Dichlorid als farbloser, kristalliner Feststoff (Ausbeute 89 %) mit einem Schmelzpunkt: von 79,5 - 80,0 °C. Furan-2,5-dicarbonsäuredichlorid wurde bis zur Weiterverwendung unter Schutzgas (Argon) im Dunklen bei Raumtemperatur gelagert.

### Herstellung der Furan-2,5-dicarbonsäureester aus Furan-2,5-dicarbonsäuredichlorid (II)

Unter Argon wurde in einem Dreihalskolben mit Rückflusskühler und Tropftrichter das Dichlorid vorgelegt und durch Erhitzen geschmolzen. Zur Flüssigkeit wurden 2,4 Äquivalente n-Pentanol langsam zugetropft, wobei es zur exothermen Reaktion mit Gasentwicklung kam. Das entstehende Gas wurde durch Waschflaschen mit wässriger KOH-Lösung geleitet. Nach vollständiger Zugabe wurde 16 h bei einer Temperatur von 80 - 100 °C gerührt.

Der überschüssige Alkohol wurde in Gegenwart von Siedesteinen unter reduziertem Druck entfernt und das Rohprodukt destillativ aufgereinigt. Man erhält Dipentylfuran-2,5-dicarboxylat, das für die weiteren Versuche eingesetzt wird.

### Charakterisierung des Dipentylfuran-2,5-dicarboxylates

### 1.1 Bestimmung der Ester-Reinheit mittels gaschromatographischer Analyse (GC)

Die Bestimmung der Reinheit der hergestellten Ester mittels GC erfolgt mit einem GC-Automaten "6890N" von Agilent Technologies unter Verwendung einer DB-5-Säule (Länge: 20m, Innendurchmesser: 0,25 mm, Filmdicke 0,25 µm) von J&W Scientific und einem Flammenionisationsdetektor bei folgenden Rahmenbedingungen:

| | |
|---|---|
| Starttemperatur Ofen: 150 °C | Endtemperatur Ofen: 350 °C |
| (1) Heizrate 150-300 °C: 10 K/min | (2) Isotherm : 10 min. bei 300 °C |
| (3) Heizrate 300-350 °C: 25 K/min. | |
| Gesamtlaufzeit: 27 min. | |
| | |
| Eingangstemperatur Einspritzblock: 300 °C | Splittverhältnis: 200:1 |
| Splitfluss: 512,2 ml/min | Gesamtfluß: 517,7 ml/min. |
| Trägergas: Helium | Injektionsvolumen: 3 Mikroliter |
| | |
| Detektortemperatur: 350 °C | Brenngas: Wasserstoff |
| Flußrate Wasserstoff: 40 ml/min. | Flußrate Luft: 440 ml/min. |
| Makeup-Gas: Helium | Flußrate Makeup-Gas: 45 ml/min. |

Die Auswertung der erhaltenen Gaschromatogramme erfolgt manuell gegen vorhandene Vergleichssubstanzen die Angabe der Reinheit erfolgt in Flächenprozent. Auf Grund der hohen Endgehalte an Zielsubstanz von > 98 % ist der zu erwartende Fehler durch fehlende Kalibrierung auf die jeweilige Probensubstanz gering.

Die Messung ergab eine Reinheit des unter Beispiel 1 hergestellten Esters von 98,9 Flächen%.

### 1.2 Bestimmung der Dichte des hergestellten Esters

Die Bestimmung der Dichte der hergestellten Ester erfolgt mittels Biegeschwinger gemäß DIN 51757 - Verfahren 4.

Die Messung ergab eine Dichte von 1,0468 g/cm³.

### 1.3 Bestimmung der APHA-Farbzahl des hergestellten Esters

Die Bestimmung der Farbzahl der hergestellten Ester erfolgte gemäß DIN EN ISO 6271-2.

Die Messung ergab eine APHA-Farbzahl von 41.

### 1.4 Bestimmung der Säurezahl des hergestellten Esters

Die Bestimmung der Säurezahl der hergestellten Ester erfolgte gemäß DIN EN ISO 2114.

Die Bestimmung ergab eine Säurezahl von 0,16 mg KOH/g

### 1.5 Bestimmung des Wassergehaltes des hergestellten Esters

Die Bestimmung des Wassergehaltes der hergestellten Ester erfolgte gemäß DIN 51777 Teil 1 (Direktes Verfahren).

Die Bestimmung ergab einen Wassergehalt von 0,042 %.

### 1.6 Bestimmung der Eigenviskosität des hergestellten Esters

Die Bestimmung der Eigenviskosität (Scherviskosität) des hergestellten Esters erfolgte unter Verwendung eines Physia MCR 101 (Fa. Anton-Paar) mit Z3-Meßsystem (DIN 25mm) im Rotationsmodus über folgendes Verfahren:
Ester und Meßsystem wurden zunächst auf eine Temperatur von 20 °C temperiert, anschließend wurden folgende Punkte angesteuert:
   1. Eine Vorscherung von 100 s⁻¹ für den Zeitraum von 60 s, bei der keine Messwerte aufgenommen wurden (zur Nivellierung eventuell auftretender thixotroper Effekte und zur besseren Temperaturverteilung).
   2. Eine Frequenz-Abwärtsrampe, beginnend bei 500 s⁻¹ und endend bei 10 s⁻¹, aufgeteilt in eine logarithmische Reihe mit 20 Schritten mit jeweils 5 s Messpunktdauer (Verifizierung des newtonschen Verhaltens).

Der Ester zeigte ein newtonsches Fließverhalten.

Die Messung ergab eine Scherviskosität (bei 42 s⁻¹) von 44 mPa*s.

### 1.7 Bestimmung von Glasübergangstemperatur und Schmelztemperatur des hergestellten Esters mittels DSC

Die Bestimmung der und der Glasübergangstemperatur und der Schmelztemperatur erfolgt über Differentialkalorimetrie (DSC) gemäß DIN 51007 (Temperaturbereich von -150 °C bis +200 °C) aus der ersten Aufheizkurve bei einer Heizrate von 10K/min. Der Wendepunkt der Wärmestromkurve wird als Glasübergangstemperatur ausgewertet.

Die Messung ergab eine Glasübergangstemperatur von -34 °C und eine Schmelztemperatur von +12 °C, die Probe war bei zuvor 7 Tage bei Raumtemperatur flüssig gelagert worden.

### Beispiel 2

### Lösetemperatur der Weichmacher

Die Lösetemperatur gibt an, ab welcher Temperatur ein in einem kontinuierlich aufgeheizten Weichmacherüberschuss (96 g Weichmacher 4 g Polymer) dispergiertes PVC-Pulver gelöst wird, und lässt Rückschlüsse auf das Gelierverhalten zu. Di-n-butylfuran-2,5-dicarboxylat (DNBFDC) und Di-n-hexylfuran-2,5-dicarboxylat (DNHFDC) wurden unter Verwendung von n-Butanol bzw. n-Hexanol analog zu Beispiel 1 hergestellt. Die Feststoffe Di-n-butylfuran-2,5-dicarboxylatund Di-n-hexylfuran-2,5-dicarboxylat wurden zunächst bei 50 °C aufgeschmolzen, das erfindungsgemäße Di-n-pentylfuran-2,5-dicarboxylat (DNPFDC) wurde auf 50 °C temperiert bevor das PVC-Pulver in der Flüssigkeit dispergiert und die Temperatur erhöht wurde. Versuchsdurchführung:
In einem 150 ml Becherglas werden 96 g des entsprechenden Weichmachers und 4 g des PVC's Lacovyl PB 1704 H (Fa. Arkema) eingewogen. Zu der Mischung werden ein Magnetrührfisch und ein an einem Stativ befestigtes Innenthermometer (Bereich: 0 °C - 250 °C, Anzeigegenauigkeit: 0,5 °C) gegeben. Mit einem Draht oder Klebeband wird an der Rückseite des Glases ein Papierstreifen, der den Schriftzug "Lösetemperatur" in der Schriftart "Times New Roman", Schriftgröße 12, so befestigt, dass der Schriftzug durch das Becherglas hindurch zu sehen ist. Danach wird die Heizplatte eines beheizbaren Laborrührgeräts (MR-Hei-Standard) auf 200 °C und die Drehzahl auf 600 U/min eingestellt. Nach Erreichen einer Innentemperatur der Flüssigkeit von 140 °C wurde die Solltemperatur nochmals auf 250 °C hochgeregelt. Die Lösetemperatur ist dann erreicht, wenn der Schriftzug durch die Flüssigkeit gerade klar lesbar geworden ist

Für DNBFDC, DNPFDC und DNHFDC wurden folgende Werte ermittelt (Doppelbestimmung), die in Tabelle 1 dargestellt sind:

**Tabelle 1: Ergebnisse der Versuche zur Lösetemperatur.**

| Weichmacher | Lösetemperatur [°C] |
|---|---|
| DNBFDC | 117 |
| DNPFDC* | 96 |
| DNHFDC | 104 |

| | |
|---|---|
| * erfindungsgemäß | |

Völlig überraschend liegt die Lösetemperatur des erfindungsgemäßen DNPFDC deutlich unterhalb des homologen DNBFDC. Wie die nachfolgenden Versuche zeigen, weist DNPFDC ein deutlich besseres Geliervermögen auf.

### Beispiel 3 Verwendung der erfindungsgemäßen Furandicarbonsäuredipentylester in einem PVC Plastisol zur Herstellung von Deckstrichfilmen für Fußbodenbeläge: Herstellung der Plastisole

Die mit den erfindungsgemäßen Weichmachern erzielbaren vorteilhaften Eigenschaften sollen im Folgenden an Plastisolen/Pasten aufgezeigt werden, wie sie beispielsweise zur Herstellung einer transparenten Deckschicht (sog. "Deckstrich") in mehrschichtig aufgebauten Fußbodenbelägen verwendet werden. Die verwendeten Einwaagen in Gramm der Komponenten für die verschiedenen Pasten sind der nachfolgenden Tabelle 2 zu entnehmen. Beispiel 3 und 6 sind erfindungsgemäß, die anderen Beispiele 1, 2, 4, 5, 7 und 8 sind Vergleichsbeispiele.

**Tabelle 2: Rezepturen (Alle Angaben in Massenteilen)**

| Pastenrezeptur | 1 | 2 | 3* | 4 | 5 | 6* | 7 | 8 |
|---|---|---|---|---|---|---|---|---|
| Vestolit B 7021 - Ultra | 100 | 100 | 100 | 100 | 100 | 100 | 100 | 100 |
| Vestinol® 9 | 50 | | | | 25 | 25 | 25 | |
| DNBFDC | | 50 | | | 25 | | | |
| DNPFDC | | | 50 | | | 25 | | |
| DNHFDC | | | | 50 | | | 25 | |
| Eastman DBT | | | | | | | | 50 |
| Drapex 39 | 3 | 3 | 3 | 3 | 3 | 3 | 3 | 3 |
| Mark CZ 149 | 2 | 2 | 2 | 2 | 2 | 2 | 2 | 2 |

| | | | | | | | | |
|---|---|---|---|---|---|---|---|---|
| * = erfindungsgemäß | | | | | | | | |

Die verwendeten Substanzen werden im Folgenden beschrieben:
Vestolit B 7021 - Ultra: Mikrosuspensions-PVC (Homopolymer) mit einem K-Wert (bestimmt gemäß DIN EN ISO 1628-2) von 70; Fa. Vestolit GmbH.
Vestinol® 9 :Diisononyl(ortho)phthalat (DINP), Weichmacher; Fa. Evonik Oxeno GmbH.
DNBFDC: Di-n-butylfuran-2,5-dicarboxylat (Hergestellt mit n- Butanol analog Beispiel 1)
DNPFDC: Erfindungsgemäßer Furandicarbonsäuredipentylester gemäß Bsp. 1
DNHFDC: Di-n-hexylfuran-2,5-dicarboxylat (Hergestellt mit n- Hexanol analog Beispiel 1)
Eastman DBT: Dibutylterephthalat, Weichmacher mit schneller Gelierung, Fa. Eastman Chemicals)
Drapex 39 : Epoxidiertes Sojabohnenöl; Costabilisator mit weichmachender Wirkung; Fa. Galata Chemicals.
Mark CZ 149: Ca/Zn-Stabilisator, Fa. Galata Chemicals

Die flüssigen Bestandteile wurden vor den festen Bestandteilen in einem PE-Becher eingewogen. Die Mischung wurde mit einem Salbenspatel so eingerührt, dass kein unbenetztes Pulver mehr vorhanden war. Der Mischbecher wurde dann in die Klemmvorrichtung eines Dissolverrührers eingespannt. Mit einer Mischerscheibe wurde die Probe homogenisiert. Dabei wurde die Drehzahl von 330 U/min bis 2000 U/min erhöht, und so lange gerührt, bis die Temperatur an der Digitalanzeige des Thermofühlers 30,0 °C erreichte. Damit war sicher gestellt, dass die Homogenisierung der Paste bei einem definierten Energieeintrag erreicht wurde. Danach wurde die Paste sofort bei 25,0 °C temperiert.

Die Rezepturen 2 und 4 ließen sich nur durch Erwärmen der als Feststoff vorliegenden Furandicarboxylate herstellen. Im Anschluss an die Herstellung verfestigten sich die Pasten jedoch so stark, dass keine weitere Verarbeitung durchgeführt werden konnte.

Paste 6 zeigt die Wirkungsweise eines erfindungsgemäßen Di-n-pentyl-furandicarboxylats in Abmischungen mit einem weiteren Weichmacher.

### Beispiel 4 Verwendung der erfindungsgemäßen Furandicarbonsäuredipentylester in einem PVC Plastisol zur Herstellung von Deckstrichfilmen für Fußbodenbeläge: Messung der Pastenviskosität

Die Messung der Viskositäten der in Beispiel 3 hergestellten Pasten wurden mit einem Rheometer Physica MCR 101 (Fa. Anton Paar), wie folgt durchgeführt.

Die nach der Herstellung für 24h bei 25 °C gelagerte Paste wurde nochmals mit einem Spatel homogenisiert und in dem Messsystem Z3 (Durchmesser 25 mm) gemäß Bedienungsanleitung vermessen. Die Messung wurde isotherm bei 25 °C durchgeführt. Folgende Punkte wurden angesteuert.

Eine Vorscherung von 100 s⁻¹ für den Zeitraum von 60 s, bei der keine Messwerte aufgenommen wurden (Nivellierung thixotroper Effekte).

Eine isotherme Abwärtsrampe, beginnend bei einer Schergeschwindigkeit von 200 s⁻¹ bis herunter zu 0,1 s⁻¹, aufgeteilt in eine logarithmische Reihe mit 30 Schritten mit jeweils 5 s Messpunktdauer. Die Ergebnisse der Messung sind in Tabelle 3 dargestellt.

**Tabelle 3: Ergebnisse der Viskositätsmessungen (Schergeschwindigkeitsprofil)**

| Plastisolrezeptur gemäß Bsp. 3 | 1 | 2 | 3* | 4 | 5 | 6* | 7 | 8 |
|---|---|---|---|---|---|---|---|---|
| Scherviskosität bei Schergeschwindigkeit= 100/s [Pa*s] | 9,8 | n.bb. | 15,8 | n.bb. | 8,1 | 6,9 | 6,5 | 3,9 |
| Scherviskosität bei Schergeschwindigkeit= 10/s [Pa*s] | 4,7 | n.bb. | 10,3 | n.bb. | 4,6 | 3,7 | 3,6 | 2,2 |
| Scherviskosität bei Schergeschwindigkeit= 1/s [Pa*s] | 4,6 | n.bb. | 10 | n.bb. | 4,4 | 3,5 | 3,5 | 2,4 |
| Scherviskosität bei Schergeschwindigkeit= 0,1/s [Pa*s] | 3,4 | n.bb. | 13,4 | n.bb. | 5,5 | 4,4 | 4,4 | 3,4 |
| Schwankungsbreite [%] (Max. Visko - Min. Visko / Min. Visko)*100 | 188 | n.bb. | 58 | n.bb. | 84 | 97 | 85 | 77 |

| | | | | | | | | |
|---|---|---|---|---|---|---|---|---|
| n.bb.: Nicht bestimmbar; Paste stark verfestigt; Messung nicht möglich. *= erfindungsgemäß | | | | | | | | |

Im Vergleich der Pasten, die nur eine Weichmachersubstanz enthalten (Pasten 1, 2, 3, 4, 8) weist die erfindungsgemäße Paste mit Abstand die geringste Schwankungsbreite der Pastenviskosität auf. Dieses Ergebnis ist insbesondere im Vergleich zwischen dem erfindungsgemäßen Furandicarbonsäuredipentylester und dem strukturell ähnlichen Dibutylterephthalat überraschend. Eine Abmischung des Standardweichmachers Vestinol 9 mit dem erfindungsgemäßen Furandicarbonsäuredipentylester (Paste 6) führt zu einer sehr starken Reduktion der Schwankungsbreite im Vergleich mit einer Paste welche allein Vestinol 9 als Weichmacher enthält (Paste 1). Das generell erhöhte Viskositätsniveau der erfindungsgemäßen Pasten kann vom Fachmann leicht, z.B. durch Zusatz von Viskositätsadditiven oder Lösungsmitteln an die bei der jeweiligen Verarbeitungsart vorliegenden Gegebenheiten angepasst werden.

### Beispiel 5 Verwendung der erfindungsgemäßen Furandicarbonsäuredipentylester in einem PVC Plastisol zur Herstellung von Deckstrichfilmen für Fußbodenbeläge: Bestimmung des Gelierverhaltens (Geliergeschwindigkeit)

Die Untersuchung des Gelierverhaltens der Plastisole wurde im Physica MCR 101 im Oszillationsmodus mit einem Platte-Platte Meßsystem (PP25), welches schubspannungsgesteuert betrieben wurde, vorgenommen. Eine zusätzliche Temperierhaube wurde an das Gerät angeschlossen, um eine homogene Wärmeverteilung zu erreichen.

### Meßparameter:

Modus: Temperatur-Gradient (Temperaturrampe)
Start-Temperatur: 25 °C
End-Temperatur: 180 °C
Heiz/Kühlrate:5 K/min
Oszillations-Frequenz: 4 - 0,1 Hz Rampe (logarithmisch)
Kreisfrequenz Omega: 10 1/s
Anzahl Messpunkte: 63
Messpunktdauer: 0,5 min
Automatische Spaltnachführung F : 0 N
konstante Messpunktdauer
Spaltweite 0,5 mm

### Durchführung der Messung:

Auf die untere Messsystemplatte wurde ein Tropfen der zu messenden Plastisolrezeptur luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Temperierhaube über der Probe positioniert und die Messung gestartet.
Bestimmt wurde die sog. komplexe Viskosität des Plastisols in Abhängigkeit von der Temperatur. Ein Einsetzen des Geliervorganges war in einem plötzlichen starken Anstieg der komplexen Viskosität zu erkennen. Je früher dieser Viskositätsanstieg einsetzte, desto besser war die Gelierfähigkeit des Systems.

Aus den erhaltenen Meßkurven wurden durch Interpolation für jedes Plastisol die Temperaturen bestimmt, bei der eine komplexe Viskosität von 1000 Pa * s bzw. 10.000 Pa*s erreicht war. Zusätzlich wurde mittels Tangentenmethode die im vorliegenden Versuchsaufbau maximal erreichte Plastisolviskosität bestimmt, sowie durch Fällen eines Lotes die Temperatur, ab der die maximale Plastisolviskosität auftritt. Die Ergebnisse sind in Tabelle 4 zusammengestellt.

**Tabelle 4:Ergebnisse der Gelierversuche**

| Plastisolrezeptur (gemäß Bsp. 3) | 1 | 2 | 3* | 4 | 5 | 6* | 7 | 8 |
|---|---|---|---|---|---|---|---|---|
| Erreichen einer Plastisolviskosität von 1.000 Pa*s bei [°C] | 87 | n.bb. | 63 | n.bb. | 68 | 72 | 75 | 65 |
| Erreichen einer Plastisolviskosität von 10.000 Pa*s bei [°C] | 97 | n.bb. | 66 | n.bb. | 73 | 76 | 80 | 68 |
| Maximale Plastisolviskosität [Pa*s] | 26.900 | n.bb. | 120.000 | n.bb. | 71.900 | 63.000 | 46.700 | 91.400 |
| Temperatur beim Erreichen der maximalen Plastisolviskosität [°C] | 137 | n.bb. | 82 | n.bb. | 85 | 87 | 90 | 83 |

| | | | | | | | | |
|---|---|---|---|---|---|---|---|---|
| n.bb.: Nicht bestimmbar; Paste stark verfestigt; Messung nicht möglich. *= erfindungsgemäß | | | | | | | | |

Im Vergleich der Pasten, die nur eine Weichmachersubstanz enthalten (Pasten 1, 2, 3, 4, 8) weist die erfindungsgemäße Paste die schnellste Gelierung (d.h. hier auch Gelierung bei den niedrigsten Temperaturen) auf. Im Vergleich mit dem Standardweichmacher Vestinol® 9 erweist sich das erfindungsgemäße Di-n-pentyl-2.5-furandicarboxylat (DNPFDC) als "Schnellgelierer", also als Verbindung welche im Vergleich mit Standardweichmachern eine Gelierfähigkeit bei deutlich niedrigeren Temperaturen besitzt. Die DNPFDC-Paste geliert sogar etwas schneller als die Paste welche Eastman DBT enthält, eine Substanz mit ähnlicher Struktur und kürzeren Esterketten. Dies ist umso mehr überraschend, da die Gelierfähigkeit bei aromatischen Dicarbonsäureestern (z.B. Orthophthlate, Terephthalate usw.) im Allgemeinen mit sinkender Kettenlänge zunimmt. Durch Abmischung der erfindungsgemäßen Furandicarbonsäuredipentylesters mit dem Standardweichmacher Vestinol 9 wird eine (im Vergleich zum reinen Vestinol® 9) starke Beschleunigung des Geliervorganges (insbesondere im Hinblick auf die Temperatur bei welcher die Maximalviskosität erreicht wird) ermöglicht.

Eindeutig zu erkennen ist auch, dass der Geliervorgang der erfindungsgemäßen Paste 3 zu einer sehr viel höheren Endviskosität führt, als dies bei allen anderen Pasten der Fall ist. Die Verwendung des erfindungsgemäßen Furandicarbonsäure Dipentylesters führt im ausgelierten Zustand somit auch zu herausragenden Materialeigenschafften (insbesondere Festigkeiten) beim hergestellten Halb- bzw. Fertigzeug/Formkörper.

### Beispiel 6: Verwendung der erfindungsgemäßen Furandicarbonsäuredipentylester in einem PVC Plastisol zur Herstellung von Deckstrichfilmen für Fußbodenbeläge: Bestimmung der Shore A Härte von Gießlingen (Weichmachereffizienz)

Die Shore-Härte ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore-Härte. Da in der Praxis Formulierungen / Rezepturen häufig auf eine bestimmte Shorehärte hin eingestellt bzw. optimiert werden, kann bei sehr effizienten Weichmachern demnach ein bestimmter Anteil in der Rezeptur eingespart werden, was z.B. eine Kostenreduktion für den Verarbeiter bedeutet.

Zur Bestimmung der Shore-Härten wurden die gemäß Beispiel 3 hergestellten Plastisole in runde Gießformen aus Messing mit einem Durchmesser von 42 mm gegossen (Einwaage: 20,0 g). Dann wurden die Plastisole in den Formen im Umlufttrockenschrank 30 min bei 200 °C geliert, nach Abkühlung entnommen und vor der Messung mindestens 24 Stunden im Trockenschrank (25 °C) gelagert. Die Dicke der Scheiben betrug ca. 12 mm. Die Ergebnisse der Härtebestimmung sind in Tabelle 5 zusammengestellt.

**Tabelle 5:**

| Plastisolrezeptur (gemäß Bsp. 3) | 1 | 2 | 3* | 4 | 5 | 6* | 7 | 8 |
|---|---|---|---|---|---|---|---|---|
| Shore A | 82 | n.bb. | 72 | n.bb. | 75 | 76 | 77 | 73 |

| | | | | | | | | |
|---|---|---|---|---|---|---|---|---|
| n.bb.= Nicht bestimmbar; Paste stark verfestigt; kein Gießling herstellbar. *= erfindungsgemäß | | | | | | | | |

Im Vergleich der Pasten, die nur eine Weichmachersubstanz enthalten (Pasten 1, 2, 3, 4, 8) weist der aus der erfindungsgemäßen Paste 3 hergestellte Gießling die niedrigste Shore A-Härte und auf, und ist damit "am weichsten". D.h. auch, die Effizienz des erfindungsgemäßen Furandicarbonsäuredipentylester hinsichtlich der Weichmachung von PVC in der Paste ist überraschender Weise am höchsten, auch höher als die des Dibutyleterphthalates (Paste 8). Dies ist insofern erstaunlich, als -ähnlich wie die Gelierfähigkeit- die weichmachende Wirkung bei aromatischen Dicarbonsäureestern (z.B. Orthophthlate, Terephthalate usw.) im Allgemeinen mit sinkender Kettenlänge zunimmt.

### Beispiel 7: Verwendung der erfindungsgemäßen Furandicarbonsäuredipentylester in einem PVC Plastisol zur Herstellung von Deckstrichfilmen für Fußbodenbeläge: Bestimmung von Transparenz und Gelbwert der transparenten Deckstrichfolien

Die Herstellung der Folien erfolgte nach einer Reifezeit der Plastisole von 24 Stunden (bei 25 °C). Für die Folienherstellung wurde am Rollrakel eines Mathis Labcoaters (Hersteller: Fa. W. Mathis AG) ein Rakelspalt von 1,40 mm eingestellt. Dieser wurde mit einer Fühlerblattlehre kontrolliert und ggf. nachgestellt. Die hergestellten Pasten wurden auf ein in einem Rahmen plan eingespanntes Hochglanzpapier (Ultracast Patent; Fa. Sappi Ltd.) mittels dem Rollrakel des Mathis Labcoaters aufgerakelt. Das aufgerakelte Plastisol wurde nun für 2 min im Mathis Ofen bei 200 °C ausgeliert. Nach Abkühlung wurde die Foliendicke mit Hilfe eines Dickenschnellmessers (KXL047; Fa. Mitutoyo) mit einer Genauigkeit von 0,01 mm bestimmt. Die Foliendicke dieser Folie betrug bei dem angegebenen Rakelspalt in allen Fällen zwischen 0,95 und 1,05 mm. Die Messung der Dicke wurde an drei unterschiedlichen Stellen der Folie durchgeführt.

Die Transparenz ist ein wesentliches Kriterium zur Qualitätsbeurteilung von PVC-Deckstrichen im Fußbodenbereich, da nur bei hoher Transparenz (= geringer Opazität) ein optimales Gesamterscheinungsbild erzielt werden kann. Die Transparenz einer PVC-Deckstrichfolie gilt auch als Maß für die Verträglichkeit der zur Folienherstellung verwendeten Rezepturbestandteile, insbesondere als Maß zur Bewertung der Verträglichkeit von PVC-Matrix und Weichmacher. Hohe Transparenz (= geringe Opazität) bedeutet in der Regel eine gute Verträglichkeit. Die Bestimmung der Opazität erfolgte mit einem "Spectro Guide" Gerät der Fa. Byk Gardner. Als Hintergrund für die Opazitätsmessungen wurden eine weiße Kachel und eine schwarze Kachel benutzt. Die Messungen wurden an 3 unterschiedlichen Stellen der Proben durchgeführt und automatisch ausgewertet (Durchschnittswert).

Der Gelbwert ist ein weiteres wichtiges Qualitätskriterium. Eine Gelbfärbung im Deckstrich kann zu einer erheblichen optischen Beeinträchtigung eines Fußbodendekors führen, weshalb beim PVC-Deckstrich in der Regel nur sehr geringe Gelbwerte toleriert werden können. Die Gelbverfärbung kann einerseits durch Rezepturbestandteile (wie auch durch ihre Neben- und Abbauprodukte) hervorgerufen werden, andererseits durch (z.B. thermooxidativen) Abbau während des Herstellprozesses und/oder während der Verwendung des Deckstriches bzw. des Fußbodenbelages auftreten.

Der Gelbwert (Index YD 1925) ist ein Maß für Gelbverfärbung eines Probekörpers. Die Farbmessung erfolgte mit einem "Spectro Guide" Gerät der Fa. Byk-Gardner. Als Hintergrund für die Farbmessungen wurde eine weiße Referenz-Kachel benutzt. Folgende Parameter wurden eingestellt:
Lichtart: C/2°
Anzahl Messungen:3
Anzeige: CIE L*a*b*
Messindex: YD1925

Die Messungen selbst wurden an 3 unterschiedlichen Stellen der Proben durchgeführt. Die Werte aus den 3 Messungen wurden gemittelt. Tabelle 6 zeigt die Ergebnisse.

**Tabelle 6: Opazität und Gelbwerte transparenter Deckstrichfolien**

| Plastisolrezeptur (gemäß Bsp. 3) | 1 | 2 | 3* | 4 | 5 | 6* | 7 | 8 |
|---|---|---|---|---|---|---|---|---|
| Opazität [-] | 10,2 | - | 10,6 | - | 10,4 | 10,3 | 10,1 | 12,1 |
| Gelbwert [-] | 9,4 | - | 13,0 | - | 10,7 | 10,7 | 8,8 | 8,9 |

| | | | | | | | | |
|---|---|---|---|---|---|---|---|---|
| *= erfindungsgemäß | | | | | | | | |

Beispiele 2 und 4 konnten nicht ermittelt werden, weil die Plastisole zu hart (d.h. ungeeignet) waren, um Deckstrichfolien herzustellen. Im Vergleich der Einzelsubstanzen (1, 3 und 8) zeigt die für die aus der erfindungsgemäßen DNPFDC -Paste (3) hergestellte Deckstrichfolie, eine sehr hohe Transparenz (geringe Opazität) insbesondere im Vergleich mit der auf Basis Eastman DBT hergestellten. Dies ist insofern erstaunlich, als -ähnlich wie die Gelierfähigkeit- die Verträglichkeit des Weichmachers mit der PVC-Matrix (ausschlaggebend für die Transparenz) bei aromatischen Dicarbonsäureestern (z.B. Orthophthlate, Terephthalate usw.) im Allgemeinen mit sinkender Kettenlänge zunimmt, da gleichzeitig der unpolare Anteil abnimmt. Besonders bemerkenswert ist der geringe Unterschied zum Standardweichmacher DINP (1), der auf eine ausgezeichnete Verträglichkeit des erfindungsgemäßen DNPFDC mit der PVC-Matrix hindeutet. Der leicht erhöhte Gelbwert bei der aus dem erfindungsgemäßen DNPFDC hergestellten Deckstrichfolie, ist auf die zur Herstellung des erfindungsgemäßen Produktes verwendeten nachwachsenden Rohstoffe (Zucker bzw. Kohlenhydratderivate) zurückzuführen, und lässt sich vom Fachmann durch weitere Optimierung des Herstellverfahrens oder Auswahl eines (zusätzlichen) optimierten Stabilisators einfach herabsetzen.

### Beispiel 8 Verwendung der erfindungsgemäßen Furandicarbonsäuredipentylester in einem PVC Plastisol zur Herstellung von Polymerschäumen für Fußbodenbeläge: Herstellung der Plastisole

Im folgenden Beispiel werden Mischungen der erfindungsgemäßen Furandicarbonsäuredipentylester mit anderen Weichmachern in Formulierungen vorgestellt, wie sie z.B. für die Herstellung mehrschichtig aufgebauter Fußbodenbeläge verwendet werden. Es wurden Pasten mit der Zusammensetzung wie in der nachfolgenden Tabelle 7 angegeben hergestellt. Die Herstellung der Pasten erfolgte analog zu der in Beispiel 3 dargestellten Vorgehensweise.

**Tabelle 7: Pastenrezepturen (Alle Angaben in Massenteilen)**

| | 1 | | | | | |
|---|---|---|---|---|---|---|
| | | 2 | 3 | 4 | 5 | 6 |
| Pasten | | | | | | |
| Vinnolit MP 6852 | 100 | 100 | 100 | 100 | 100 | 100 |
| Vestinol® 9 | 59 | | | | | |
| DINFDC | | 54 | 49 | 44 | | |
| DINT | | | | | 44 | |
| Hexamoll ® DINCH | | | | | | 44 |
| DNPFDC * | | 5 | 10 | 15 | 15 | 15 |
| Unifoam AZ Ultra 7043 | 3 | 3 | 3 | 3 | 3 | 3 |
| ZnO Batch 1:2 DINP | 2 | 2 | 2 | 2 | 2 | 2 |

| | | | | | | |
|---|---|---|---|---|---|---|
| * = erfindungsgemäßer Weichmacher | | | | | | |

Die verwendeten Substanzen werden im Folgenden näher erläutert:
Vinnolit MP 6852: Mikrosuspensions-PVC (Homopolymer) mit K-Wert (gemäß DIN EN ISO 1628-2) von 68; Fa. Vinnolit GmbH & Co KG.
Vestinol® 9: Diisononyl(ortho)phthalat (DINP), Weichmacher; Fa. Evonik Oxeno GmbH. Unifoam AZ Ultra 7043: Azodicarbonamid; thermisch aktivierbares Treibmittel; Fa. Hebron S.A.
Zinkoxid: ZnO; Zersetzungskatalysator für thermisches Treibmittel; setzt die substanzeigene Zersetzungstemperatur des Treibmittels herab; wirkt gleichzeitig auch als Stabilisator; "Zinkoxid aktiv®"; Fa. Lanxess AG. Das Zinkoxid wurde mit einer ausreichenden (Teil)Menge des jeweils verwendeten Weichmachers vorvermischt und anschließend zugegeben.
DINFDC: Diisononylfurandicarbonsäureester, Laborprodukt; Herstellung analog Beispiel 1 jedoch unter Verwendung von Isononylalkohol (Fa. Evonik Oxeno GmbH)
DINT: Diisononylterephthalat; Laborprodukt; hergestellt gemäß WO 2009/095126 A1 aus Isononylalkohol (Fa. Evonik Oxeno GmbH)
Hexamoll ® DINCH: Diisononylcyclohexancarbonsäureester; Fa.BASF SE
DNPFDC: Di-n-pentylfuran-2,5-dicarboxylat gemäß Bsp. 1

### Beispiel 9 Verwendung der erfindungsgemäßen Furandicarbonsäuredipentylester in einem PVC Plastisol zur Herstellung von Polymerschäumen für Fußbodenbeläge: Bestimmung der Pastenviskosität

Die Bestimmung der Pastenviskosität erfolgte wie in Beispiel 4 beschrieben, die Ergebnisse sind in Tabelle 8 dargestellt.

**Tabelle 8:**

| Plastisolrezeptur gemäß Bsp. 8 | 1 | 2* | 3* | 4* | 5* | 6* |
|---|---|---|---|---|---|---|
| Scherviskosität bei Schergeschwindigkeit= 100/s [Pa*s] | 3,1 | 6,4 | 6,6 | 7,1 | 3,3 | 2,6 |
| Scherviskosität bei Schergeschwindigkeit= 10/s [Pa*s] | 2,5 | 5,3 | 5,6 | 6,1 | 2,6 | 2,3 |
| Scherviskosität bei Schergeschwindigkeit= 1/s [Pa*s] | 2,9 | 5,8 | 6,2 | 6,9 | 2,9 | 2,9 |
| Scherviskosität bei Schergeschwindigkeit= 0,1/s [Pa*s] | 4,3 | 8,1 | 8,8 | 10,1 | 4,2 | 4,7 |

| | | | | | | |
|---|---|---|---|---|---|---|
| *= erfindungsgemäß | | | | | | |

Die Ergebnisse zeigen, dass insbesondere Mischungen mit Weichmachern wie dem Terephthalat DINT und mit DINCH zu niedrigen Pastenviskositäten und sehr guten Verarbeitungseigenschaften führen. Diese sind deutlich besser als für das Vergleichsprodukt Vestinol® 9, insbesondere durch Abmischung mit DINCH lässt sich eine sehr niedrige Pastenviskosität einstellen, die auch für sehr schnell laufende Verarbeitungverfahren (z.B. bei Rakelauftrag) geeignet ist. Diese Abmischungen vereint besonders vorteilhaft eine gute Verarbeitbarkeit, die Freiheit von Phthalaten und eine besonders hohe Nachhaltigkeit (im Hinblick auf die Rohstoffbasis der erfindungsgemäßen Furandicarbonsäuredipentylester).

### Beispiel 10: Verwendung der erfindungsgemäßen Furandicarbonsäuredipentylester in einem PVC Plastisol zur Herstellung von Polymerschäumen für Fußbodenbeläge: Bestimmung der Geliereigenschaften (Geliergeschwindigkeit)

Die Bestimmung der Geliereigenschaften erfolgte wie in Beispiel 5 beschrieben, jedoch an den gemäß Beispiel 8 hergestellten Plastisolen. Die Ergebnisse sind in Tabelle 9 dargestellt.

**Tabelle 9:**

| Plastisolrezeptur (gemäß Bsp. 8) | 1 | 2* | 3* | 4* | 5* | 6* |
|---|---|---|---|---|---|---|
| Erreichen einer Plastisolviskosität von 1.000 Pa*s bei [°C] | 83 | 78 | 76 | 74 | 81 | 78 |
| Erreichen einer Plastisolviskosität von 10.000 Pa*s bei [°C] | 88 | 82 | 80 | 78 | 97 | 83 |
| Maximale Plastisolviskosität [Pa*s] | 26.300 | 32.500 | 38.300 | 46.600 | 23.100 | 22.600 |
| Temperatur beim Erreichen der maximalen Plastisolviskosität [°C] | 130 | 100 | 90 | 86 | 128 | 125 |

| | | | | | | |
|---|---|---|---|---|---|---|
| * = erfindungsgemäß | | | | | | |

Im Vergleich mit dem Standardweichmacher Vestinol® 9 zeigen alle Abmischungen der erfindungsgemäßen Furandicarbonsäureester ein deutlich verbessertes Gelierverhalten mit Gelierung bei teilweise deutlich niedrigen Temperaturen. Mit zunehmender Konzentration an erfindungsgemäßem Ester nimmt die Geliergeschwindigkeit und die maximal erreichbare Viskosität deutlich zu bzw. die Geliertemperatur deutlich ab. Durch Abmischung des (sehr langsam gelierenden) DINCH (Paste 5) mit dem erfindungsgemäßen Ester wird ein Gelierverhalten eingestellt, welches dem des Standardweichmachers Vestinol® 9 sehr ähnlich ist. Durch die Zugabe des erfindungsgemäßen Furandicarbonsäureesters wird somit die Verarbeitbarkeit deutlich verbessert, insbesondere lassen sich solche Pasten schneller bzw. bei niedrigeren Temperaturen verarbeiten.

### Beispiel 11 Verwendung der erfindungsgemäßen Furandicarbonsäuredipentylester in einem PVC Plastisol zur Herstellung von Polymerschäumen für Fußbodenbeläge: Bestimmung des Aufschäumverhaltens

Das Aufschäumverhalten wurde mit Hilfe eines Dickenschnellmessers mit Eignung für Weich-PVC Messungen (KXL047, Fa. Mitutoyo) mit einer Genauigkeit von 0,01 mm bestimmt. Für die Folienherstellung wurde am Rollrakel eines Mathis Labcoaters (Typ: LTE-TS; Hersteller: Fa. W. Mathis AG) ein Rakelspalt von 1 mm eingestellt. Dieser wurde mit einer Fühlerblattlehre kontrolliert und ggf. nachgestellt. Die Pasten wurden auf ein in einem Rahmen plan eingespanntes Trennpapier (Warran Release Paper; Fa. Sappi Ltd.) mittels dem Rollrakel des Mathis Labcoaters aufgerakelt. Um die prozentuale Aufschäumung errechnen zu können, wurde bei 200 °C / 30 Sekunden Verweilzeit zunächst eine angelierte und nicht geschäumte Folie hergestellt. Die Foliendicke (= Ausgangsdicke) dieser Folie betrug bei dem angegebenen Rakelspalt in allen Fällen zwischen 0,74 und 0,77 mm. Die Messung der Dicke wurde an drei unterschiedlichen Stellen der Folie durchgeführt. Anschließend wurden ebenfalls mit dem bzw. im Mathis-Labcoater die geschäumten Folien (Schäume) bei einer Ofen-Verweilzeiten (60s, 90s, 120s und 150s) hergestellt. Nach Abkühlung der Schäume wurden die Dicken ebenfalls an drei unterschiedlichen Stellen vermessen. Der Mittelwert der Dicken und die Ausgangsdicke wurden für die Berechnung der Expansion benötigt. (Beispiel: (Schaumdicke - Ausgangsdicke) / Ausgangsdicke * 100% = Expansion). Die Ergebnisse sind in Tabelle 10 dargestellt.

**Tabelle 10:Expansionsverhalten der Schaumfolien.**

| Plastisolrezeptur (gemäß Bsp. 8) | 1 | 2* | 3* | 4* | 5* | 6* |
|---|---|---|---|---|---|---|
| Expansion nach 60 s @ 200°C [%] | 22 | 8 | 8 | 8 | 8 | 1 |
| Expansion nach 90 s @ 200°C [%] | 305 | 299 | 278 | 292 | 285 | 265 |
| Expansion nach 120 s @ 200°C [%] | 386 | 414 | 407 | 414 | 407 | 393 |
| Expansion nach 150 s @ 200°C [%] | 407 | 441 | 414 | 427 | 414 | 407 |

| | | | | | | |
|---|---|---|---|---|---|---|
| * = erfindungsgemäß | | | | | | |

Die Verschäumung der Pasten, die erfindungsgemäßen Furandicarbonsäureester enthalten verläuft im Vergleich mit dem Standardweichmacher Vestinol ® 9 deutlich schneller und führt zu höheren Schaumhöhen. Dies gilt auch für die Abmischung mit DINCH und dem Terephthalat DINT.

### Beispiel 12: Verwendung der erfindungsgemäßen Furandicarbonsäuredipentylester in einem Gemisch mit zusätzlichen Weichmachern zur Herstellung von Planen: Herstellung der Plastisole

Im Folgenden sollen die Vorteile der erfindungsgemäßen Pasten an Hand von Füllstoff und Pigment enthaltenden PVC-Pasten verdeutlicht werden, die sich zur Herstellung von Planen eignen (z.B. als Grundstrich für die Fließimpregnierung). In der entsprechenden Anwendung kann die vorliegende Rezeptur einfach z.B. durch Zugabe von Haftvermittlern und/oder Flammschutzmitteln an die jeweiligen Anforderungen angepasst werden. Die Herstellung der Pasten erfolgte analog Beispiel 3 jedoch mit veränderter Rezeptur. Die verwendeten Einwaagen der Komponenten für die verschiedenen Pasten sind der nachfolgenden Tabelle 11 zu entnehmen. Alle Angaben in Massenanteilen.

**Tabelle 11: Plastisolrezepturen**

| | **1** | **2*** | **3*** | **4*** | **5*** | **6*** |
|---|---|---|---|---|---|---|
| Rezeptur: | | | | | | |
| Vestolit P 1430 K70 | 100 | 100 | 100 | 100 | 100 | 100 |
| Vestinol® 9 | 50 | | | | | |
| DINFDC | | 45 | 40 | 35 | | |
| DINT | | | | | 35 | |
| Hexamoll ® DINCH | | | | | | 35 |
| DNPFDC | | 5 | 10 | 15 | 15 | 15 |
| Calcilit 6G | 15 | 15 | 15 | 15 | 15 | 15 |
| Kronos 2220 | 3 | 3 | 3 | 3 | 3 | 3 |
| Drapex 39 | 3 | 3 | 3 | 3 | 3 | 3 |
| Mark BZ 561 | 1,5 | 1,5 | 1,5 | 1,5 | 1,5 | 1,5 |

| | | | | | | |
|---|---|---|---|---|---|---|
| *= erfindungsgemäß | | | | | | |

Die verwendeten Stoffe und Substanzen, welche sich nicht bereits aus den vorangegangenen Beispielen ergeben, werden im Folgenden näher erläutert:
Vestolit P 1430 K70: Mikrosuspensions-PVC der Fa. Vestolit GmbH
DNPFDC: Erfindungsgemäßer Furandicarbonsäuredipentylester gemäß Bsp. 1
Calcilit 6G: Füllstoff; Calciumcarbonat
Kronos 2220: Rutilpigment Fa. Kronos
Mark BZ 561: Ba/Zn Stabilisator Fa. Galata Chemicals

### Beispiel 13 Verwendung der erfindungsgemäßen Furandicarbonsäuredipentylester in einem Gemisch mit zusätzlichen Weichmachern zur Herstellung von Planen: Bestimmung der Plastisolviskosität

Die Bestimmung der Pastenviskosität erfolgte wie in Beispiel 4 beschrieben, die Ergebnisse sind in Tabelle 12 dargestellt.

**Tabelle 12: Ergebnisse der Viskositätsmessungen (Schergeschwindigkeitsprofil)**

| Plastisolrezeptur gemäß Bsp. 12 | 1 | 2* | 3* | 4* | 5* | 6* |
|---|---|---|---|---|---|---|
| Scherviskosität bei Schergeschwindigkeit= 100/s [Pa*s] | 8,9 | 15 | 14,9 | 15,8 | 9,3 | 5,7 |
| Scherviskosität bei Schergeschwindigkeit= 10/s [Pa*s] | 5,1 | 9 | 9,1 | 10,1 | 5,1 | 4 |
| Scherviskosität bei Schergeschwindigkeit= 1/s [Pa*s] | 6,3 | 9,7 | 9,9 | 11,4 | 6 | 5,1 |
| Scherviskosität bei Schergeschwindigkeit= 0,1/s [Pa*s] | 11,7 | 15,1 | 15,7 | 18,5 | 11,2 | 9,8 |

| | | | | | | |
|---|---|---|---|---|---|---|
| *= erfindungsgemäß | | | | | | |

In Abmischungen mit dem Terephthalat DINT und mit DINCH werden im Vergleich mit dem Standardweichmacher Vestinol® 9 teilweise deutlich niedrigere Plastisolviskositäten erreicht, es ist somit möglich diese Plastisole in der Streichverarbeitung deutlich schneller zu verarbeiten als beispielsweise rein auf Vestinol® 9 basierte Plastisole.

### Beispiel 14 Verwendung der erfindungsgemäßen Furandicarbonsäuredipentylester in einem Gemisch mit zusätzlichen Weichmachern zur Herstellung von Planen: Bestimmung des Gelierverhaltens

Die Bestimmung der Geliereigenschaften erfolgte wie in Beispiel 5 beschrieben, jedoch an den gemäß Beispiel 12 hergestellten Plastisolen. Die Ergebnisse sind in Tabelle 13 dargestellt.

**Tabelle 13:Ergebnisse der Gelierversuche**

| Plastisolrezeptur (gemäß Bsp. 12) | 1 | 2* | 3* | 4* | 5* | 6* |
|---|---|---|---|---|---|---|
| Erreichen einer Plastisolviskosität von 1.000 Pa*s bei [°C] | 83 | 78 | 75 | 73 | 78 | 76 |
| Erreichen einer Plastisolviskosität von 10.000 Pa*s bei [°C] | 88 | 82 | 79 | 77 | 86 | 83 |
| Maximale Plastisolviskosität [Pa*s] | 33.900 | 39.300 | 44.600 | 56.600 | 29.300 | 26.900 |
| Temperatur beim Erreichen der maximalen Plastisolviskosität [°C] | 136 | 126 | 88 | 85 | 132 | 134 |

| | | | | | | |
|---|---|---|---|---|---|---|
| * = erfindungsgemäß | | | | | | |

Die Gelierung erfolgt in allen Fällen deutlich schneller als beim Standardweichmacher Vestinol® 9. Mit steigendem Anteil an erfindungsgemäßem Furandicarbonsäuredipentylester kommt es insbesondere in Kombination mit Furandicarbonsäurediisononylester (Proben 2-4) zu einer sehr starken Beschleunigung des Geliervoganges, wie insbesondere an der Temperatur bei der die maximale Viskosität erreicht wird, zu erkennen ist. In Kombination mit dem Terephthalat DINT und mit DINCH werden Geliereigenschaften erreicht, die immer noch besser als die des Standardweichmachers Vestinol® 9 sind.

### Beispiel 15 Verwendung der erfindungsgemäßen Furandicarbonsäuredipentylester in einem Gemisch mit zusätzlichen Weichmachern zur Herstellung von Planen: Bestimmung der Weichmachereffizienz (Shore A)

Die Herstellung der Gießlinge und die Messung der Shorehärte wurden analog zu der in Beispiel 6 beschriebenen Vorgehensweise durchgeführt. Die Ergebnisse sind in Tabelle 14 dargestellt.

**Tabelle 14:Plastisolrezepturen**

| Plastisolrezeptur (gemäß Bsp. 12) | 1 | 2* | 3* | 4* | 5* | 6* |
|---|---|---|---|---|---|---|
| Shore A | 81 | 78 | 77 | 75 | 78 | 77 |

| | | | | | | |
|---|---|---|---|---|---|---|
| * = erfindungsgemäß | | | | | | |

Alle Mischungen erreichen Shore A-Werte, die unterhalb des Wertes für den Standardweichmacher Vestinol 9 liegen. Die weichmachende Wirkung aller die erfindungsgemäßen Pentylester enthaltenden Proben ist somit größer als die des Standardweichmachers. Die weichmachende Wirkung nimmt zudem eindeutig mit der Konzentration der erfindungsgemäßen Pentylester zu. Auch in Abmischungen mit dem Terephthalat DINT und mit DINCH werden niedrigere Shore A-Werte als mit Vestinol® 9 erreicht. Es bietet sich demnach für den Fachmann die Möglichkeit die Gesamtweichmachermenge durch Verwendung der erfindungsgemäßen Pentylester deutlich zu reduzieren.

## Patentansprüche

1. Verwendung von Dipentylestern der Furandicarbonsäure, deren Eigenviskosität bei 25°C maximal 60 mPa*s beträgt, als Weichmacher.

2. Verwendung gemäß Anspruch 1, **dadurch gekennzeichnet, dass** der Dipentylester zudem folgende Eigenschaft aufweist: seine Dichte bei 20 °C beträgt maximal 1,06 g/cm³.

3. Verwendung gemäß Anspruch 1, **dadurch gekennzeichnet, dass** der Dipentylester als Weichmacher für PVC verwendet wird.

4. Verwendung gemäß einem der Ansprüche 1 bis 3, **dadurch gekennzeichnet, dass** der Dipentylester ein Dipentylfuran-2,5-dicarboxylat ist.

5. Verwendung gemäß einem der Ansprüche 1 bis 4, **dadurch gekennzeichnet, dass** das Dipentylfuran-2,5-dicarboxylat in Form von mindestens zwei isomeren Dipentylfuran-2,5-dicarboxylate vorhanden ist.

6. Verwendung gemäß Anspruch 5, **dadurch gekennzeichnet, dass** die isomeren Dipentylfuran-2,5-dicarboxylate isomere Pentylgruppen enthalten.

7. Verwendung gemäß Anspruch 6, **dadurch gekennzeichnet, dass** die isomeren Pentylgruppen ausgewählt sind aus der Gruppe n-Pentyl-, 2-Pentyl-, 3-Pentyl-, 2-Methylbutyl-, 3-Methylbutyl-, 3-Methylbut-2-yl-, 2-Methylbut-2-yl-Gruppe.

8. Verwendung gemäß einem der Ansprüche 1 bis 7, **dadurch gekennzeichnet, dass** der Dipentylester der Furandicarbonsäure Teil einer Weichmacherzusammensetzung ist und die Weichmacherzusammensetzung einen zusätzlichen Weichmacher ausgewählt aus der Gruppe Alkylbenzoate, Dialkyladipate, Glycerinester, Citronensäuretrialkylester, acylierte Citronensäuretrialkylester, Trialkylmellitate, Glykoldibenzoate, Dialkylterephthalate, Dialkylphthalate, Dialkanoylester des Isosorbit, Dialkylester der 1,2-, 1,3- oder 1,4-Cyclohexandicarbonsäure enthält.

9. Verwendung gemäß einem der Ansprüche 1 oder 2 in Klebstoffen, Dichtungsmassen, Beschichtungsmassen, Lacken, Farben, Plastisolen, Pasten, Kunstleder, Fußbodenbeläge, Unterbodenschutz, Gewebebeschichtungen, Tapeten oder Tinten.

10. Kunststoffzusammensetzung enthaltend einen Dipentylester der Furandicarbonsäure, dessen Eigenviskosität bei 25°C maximal 60 mPa*s beträgt, als Weichmacher, **dadurch gekennzeichnet, dass** Weichmacher in einer Menge von 5 bis 200 Massenteilen pro 100 Massenteile Polymer enthalten ist.

11. Kunststoffzusammensetzung nach Anspruch 10, **dadurch gekennzeichnet, dass** mindestens ein Polymer enthalten ist, ausgewählt aus: Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalkohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymeren der genannten Polymere oder deren monomeren Einheiten.

12. Kunststoffzusammensetzung nach einem der Ansprüche 10 oder 11, **dadurch gekennzeichnet, dass** das mindestens eines der enthaltenen Polymere ein Copolymer von Vinylchlorid mit einem oder mehreren Monomeren ausgewählt aus der Gruppe bestehend aus Vinylidenchlorid, Vinylacetat, Vinylpropionat, Vinylbutyrat, Vinylbenzoat, Methylacrylat, Ethylacrylat, oder Butylacrylat ist.

13. Formkörper oder Folie enthaltend eine Kunststoffzusammensetzung gemäß einem der Ansprüche 10 bis 12.

14. Formkörper oder Folie nach Anspruch 13, wobei die Folie oder der Formkörper ein Fußbodenbelag, ein Wandbelag, ein Schlauch, ein Profil, eine Dachbahn, eine Dichtungsbahn, eine Kabel- oder Drahtummantelung, eine Plane, ein Werbebanner, Kunstleder, Verpackungsfolie, ein medizinischer Artikel, Spielzeug, eine Dichtung, ein Einrichtungsgegenstand ist.

## Claims

1. Use of dipentyl esters of furandicarboxylic acid having a maximum intrinsic viscosity at 25°C of not more than 60 mPa*s as a plasticizer.

2. Use according to Claim 1, **characterized in that** the dipentyl ester additionally has the following property: its density at 20°C is not more than 1.06 g/cm³.

3. Use according to Claim 1, **characterized in that** the dipentyl ester is used as a plasticizer for PVC.

4. Use according to any of Claims 1 to 3, **characterized in that** the dipentyl ester is a dipentyl furan-2,5-dicarboxylate.

5. Use according to any of Claims 1 to 4, **characterized in that** the dipentyl furan-2,5-dicarboxylate is in the form of at least two isomeric dipentyl furan-2,5-dicarboxylates.

6. Use according to Claim 5, **characterized in that** the isomeric dipentyl furan-2,5-dicarboxylates comprise isomeric pentyl groups.

7. Use according to Claim 6, **characterized in that** the isomeric pentyl groups are selected from the group of n-pentyl, 2-pentyl, 3-pentyl, 2-methylbutyl, 3-methylbutyl, 3-methylbut-2-yl, 2-methylbut-2-yl group.

8. Use according to any of Claims 1 to 7, **characterized in that** the dipentyl ester of furandicarboxylic acid is part of a plasticizer composition and the plasticizer composition comprises an additional plasticizer selected from the group of alkyl benzoates, dialkyl adipates, glyceryl esters, trialkyl citrates, acylated trialkyl citrates, trialkyl mellitates, glycol dibenzoates, dialkyl terephthalates, dialkyl phthalates, dialkanoyl esters of isosorbitol, dialkyl esters of 1,2-, 1,3- or 1,4-cyclohexanedicarboxylic acid.

9. Use according to either of Claims 1 and 2 in adhesives, sealing compounds, coating materials, lacquers, paints, plastisols, pastes, synthetic leather, floor coverings, underbody protection, fabric coatings, wallpaper or inks.

10. Polymer composition comprising a dipentyl ester of furandicarboxylic acid having a maximum intrinsic viscosity at 25°C of not more than 60 mPa*s as a plasticizer, **characterized in that** plasticizer is present in an amount of 5 to 200 parts by mass per 100 parts by mass of polymer.

11. Polymer composition according to Claim 10, **characterized in that** it comprises at least one polymer selected from polyvinyl chloride (PVC), polyvinylidene chloride (PVDC), polyacrylates, especially polymethyl methacrylate (PMMA), polyalkyl methacrylate (PAMA), fluoropolymers, especially polyvinylidene fluoride (PVDF), polytetrafluoroethylene (PTFE), polyvinyl acetate (PVAc), polyvinyl alcohol (PVA), polyvinyl acetals, especially polyvinyl butyral (PVB), polystyrene polymers, especially polystyrene (PS), expandable polystyrene (EPS), acrylonitrile-styrene-acrylate (ASA), styrene acrylonitrile (SAN), acrylonitrile-butadiene-styrene (ABS), styrene-maleic anhydride copolymer (SMA), styrene-methacrylic acid copolymer, polyolefins, especially polyethylene (PE) or polypropylene (PP), thermoplastic polyolefins (TPO), polyethylene-vinyl acetate (EVA), polycarbonates, polyethylene terephthalate (PET), polybutylene terephthalate (PBT), polyoxymethylene (POM), polyamide (PA), polyethylene glycol (PEG), polyurethane (PU), thermoplastic polyurethane (TPU), polysulphides (PSu), biopolymers, especially polylactic acid (PLA), polyhydroxybutyral (PHB), polyhydroxyvaleric acid (PHV), polyesters, starch, cellulose and cellulose derivatives, especially nitrocellulose (NC), ethylcellulose (EC), cellulose acetate (CA), cellulose acetate/butyrate (CAB), rubber or silicones, and mixtures or copolymers of the polymers mentioned or monomeric units thereof.

12. Polymer composition according to either of Claims 10 and 11, **characterized in that** at least one of the polymers present is a copolymer of vinyl chloride with one or more monomers selected from the group consisting of vinylidene chloride, vinyl acetate, vinyl propionate, vinyl butyrate, vinyl benzoate, methyl acrylate, ethyl acrylate and butyl acrylate.

13. Moulding or film comprising a polymer composition according to any of Claims 10 to 12.

14. Moulding or film according to Claim 13, wherein the film or moulding is a floor covering, a wall covering, a hose, a profile, a roofing sheet, a sealing sheet, a cable or wire sheath, a tarpaulin, an advertising banner, synthetic leather, packaging film, a medical article, a toy, a seal, a furnishing article.

## Revendications

1. Utilisation d'esters dipentyliques de l'acide furanedicarboxylique, dont la viscosité inhérente à 25 °C est au maximum de 60 mPa*s, en tant que plastifiant.

2. Utilisation selon la revendication 1, **caractérisée en ce que** l'ester dipentylique présente en outre la propriété suivante : sa densité à 20 °C est au maximum de 1,06 g/cm³.

3. Utilisation selon la revendication 1, **caractérisée en ce que** l'ester dipentylique est utilisé comme plastifiant pour PVC.

4. Utilisation selon l'une quelconque des revendications 1 à 3, **caractérisée en ce que** l'ester dipentylique est un furane-2,5-dicarboxylate de dipentyle.

5. Utilisation selon l'une quelconque des revendications 1 à 4, **caractérisée en ce que** le furane-2,5-dicarboxylate de dipentyle est présent sous forme d'au moins deux furane-2,5-dicarboxylates de dipentyle isomères.

6. Utilisation selon la revendication 5, **caractérisée en ce que** les furane-2,5-dicarboxylates de dipentyle isomères contiennent des groupes pentyle isomères.

7. Utilisation selon la revendication 6, **caractérisée en ce que** les groupes pentyle isomères sont choisis dans le groupe constitué par les groupes n-pentyle, 2-pentyle, 3-pentyle, 2-méthylbutyle, 3-méthylbutyle, 3-méthylbut-2-yle, 2-méthylbut-2-yle.

8. Utilisation selon l'une quelconque des revendications 1 à 7, **caractérisée en ce que** l'ester dipentylique de l'acide furanedicarboxylique fait partie d'une composition de plastifiant et la composition de plastifiant contient un plastifiant supplémentaire choisi dans le groupe constitué par des benzoates d'alkyle, adipates de dialkyle, esters de glycérol, esters trialkyliques d'acide citrique, esters trialkyliques d'acide citrique acylés, mellitates de trialkyle, dibenzoates de glycol, téréphtalates de dialkyle, phtalates de dialkyle, esters de dialcanoyle de l'isosorbitol, esters dialkyliques de l'acide 1,2-, 1,3- ou 1,4-cyclohexanedicarbonxylique.

9. Utilisation selon l'une quelconque des revendications 1 et 2 dans des adhésifs, des matières d'étanchéité, des matières de revêtement, des laques, des peintures, des plastisols, des pâtes, des cuirs synthétiques, des revêtements de sol, la protection de bas de caisse, des revêtements en tissu, des papiers peints ou des encres.

10. Composition de matière plastique contenant un ester dipentylique de l'acide furanedicarboxylique, dont la viscosité inhérente à 25 °C est au maximum de 60 mPa*s, en tant que plastifiant, **caractérisée en ce que** le plastifiant est contenu en une quantité de 5 à 200 parties en masse pour 100 parties en masse de polymère.

11. Composition de matière plastique selon la revendication 10, **caractérisée en ce qu'**est contenu au moins un polymère choisi parmi : le poly(chlorure de vinyle) (PVC), le poly(chlorure de vinylidène) (PVDC), des polyacrylates, en particulier le poly(méthacrylate de méthyle) (PMMA), un poly(méthacrylate d'alkyle) (PAMA), des polymères fluorés, en particulier le poly(fluorure de vinylidène) (PVDF), le polytétrafluoroéthylène (PTFE), le poly(acétate de vinyle) (PVAc), le poly(alcool vinylique) (PVA), des polyvinylacétals, en particulier le polyvinylbutyral (PVB), des polymères polystyrène, en particulier le polystyrène (PS), le polystyrène expansible (EPS), l'acrylonitrile-styrène-acrylate (ASA), le styrène-acrylonitrile (SAN), l'acrylonitrile-butadiène-styrène (ABS), un copolymère styrène-anhydride maléique (SMA), un copolymère styrène-acide méthacrylique, des polyoléfines, en particulier le polyéthylène (PE) ou le polypropylène (PP), des polyoléfines thermoplastiques (TPO), le poly(éthylène-acétate de vinyle) (EVA), des polycarbonates, le poly(éthylène-téréphtalate) (PET), le poly(butylène-téréphtalate) (PBT), le polyoxy-méthylène (POM), le polyamide (PA), le polyéthylèneglycol (PEG), le polyuréthane (PU), le polyuréthane thermoplastique (TPU), des polysulfures (PSu), des biopolymères, en particulier le poly(acide lactique) (PLA), le polyhydroxybutyral (PHB), le poly(acide hydroxyvalérique) (PHV), des polyesters, l'amidon, la cellulose et des dérivés de cellulose, en particulier la nitrocellulose (NC), l'éthylcellulose (EC), l'acétate de cellulose (CA), l'acétate/butyrate de cellulose (CAB), des caoutchoucs ou des silicones, ainsi que des mélanges ou des copolymères des polymères cités ou de leurs unités monomères.

12. Composition de matière plastique selon l'une quelconque des revendications 10 et 11, **caractérisée en ce que** ledit au moins un des polymères contenus est un copolymère de chlorure de vinyle avec un ou plusieurs monomères choisis dans le groupe constitué par le chlorure de vinylidène, l'acétate de vinyle, le propionate de vinyle, le butyrate de vinyle, le benzoate de vinyle, l'acrylate de méthyle, l'acrylate d'éthyle et l'acrylate de butyle.

13. Corps moulé ou film contenant une composition de matière plastique selon l'une quelconque des revendications 10 à 12.

14. Corps moulé ou film selon la revendication 13, le film ou le corps moulé étant un revêtement de sol, un revêtement de mur, un tuyau souple, un profilé, une bande de couverture, une bande d'étanchéité, un gainage de câble ou de fil métallique, une bâche, un bandeau publicitaire, un cuir synthétique, un film d'emballage, un article médical, un jouet, un joint d'étanchéité, un article d'ameublement.
